# EUROPEAN PATENT SPECIFICATION

(11) **EP 3 595 637 B1**
(45) Date of publication and mention of the grant of the patent: **18.08.2021**
(21) Application number: 18715811.8
(22) Date of filing: 13.03.2018
(51) Int. Cl.: A61K 9/14, A61K 9/16, A61K 9/20, A61K 9/48, A61K 31/00, A61K 36/00

(54) **COMBINATION OF ACTIVE INGREDIENTS, COMPOSITIONS COMPRISING IT AND THEIR USE IN THE TREATMENT OF SARCOPENIA**
KOMBINATION VON WIRKSTOFFEN, ZUSAMMENSETZUNGEN DAMIT UND IHRE VERWENDUNG ZUR BEHANDLUNG VON SARKOPENIE
COMBINAISON DE PRINCIPES ACTIFS, COMPOSITIONS LES COMPRENANT ET LEUR UTILISATION DANS LE TRAITEMENT DE LA SARCOPÉNIE

(30) Priority: 14.03.2017 IT 201700028228
(43) Date of publication of application: 22.01.2020
(73) Proprietor: Apharm S.r.l., 28041 Arona (NO) (IT)
(72) Inventor: PIZZONI, Angelo, I-28041 Arona (NO) (IT); PIZZONI, Paolo, I-28041 Arona (NO) (IT)
(74) Representative: Trupiano, Federica
(86) International application number: PCT/IB2018/051656
(87) International publication number: WO 2018/167661

(56) References cited:
- WO-A1-2013/188258
- US-A1- 2011 064 720
- US-A1- 2011 097 427
- ARGILÉS JOSEP M ET AL: "Skeletal Muscle Regulates Metabolism via Interorgan Crosstalk: Roles in Health and Disease", JOURNAL OF THE AMERICAN MEDICAL DIRECTORS ASSOCIATION, ELSEVIER, NL, vol. 17, no. 9, 17 June 2016 (2016-06-17), pages 789-796, XP029702493, ISSN: 1525-8610, DOI: 10.1016/J.JAMDA.2016.04.019
- M. RONDANELLI ET AL: "A Systematic Review on the Effects of Botanicals on Skeletal Muscle Health in Order to Prevent Sarcopenia", EVIDENCE-BASED COMPLEMENTARY AND ALTERNATIVE MEDICINE, vol. 2016, 1 January 2016 (2016-01-01), pages 1-23, XP055391786, ISSN: 1741-427X, DOI: 10.1155/2016/5970367

## Description

### Summary of the Invention

Subject-matter of the present invention is a novel combination of active ingredients, in particular a combination of 3-hydroxy-3-methylbutanoic acid or a pharmacologically acceptable salt thereof, a grape seed extract, a withania extract and optionally ornithine alpha-ketoglutarate. The combination of the invention is useful in the treatment of sarcopenia and in general in the diseases causing a loss of muscle mass and as anabolic agent. Further object of the invention is a pharmaceutical or nutraceutical composition comprising the combination mentioned above, together with conventional excipients and vehicles.

### Technical Field

Sarcopenia is the term used to describe the muscle mass loss, with a consequent loss of strength and physical performance, in a subject. The reasons causing sarcopenia can be various, the most common being sedentariness, long periods of physical inactivity, e.g. as a consequence of accidents or diseases, traumas involving to put in plaster parts of the body, local nervous damage, ageing and a poor or wrong diet. Alternatively, sarcopenia can be induced by diseases such as cachexia, cancer, AIDS and sepsis or can derive from a prolonged treatment with glucocorticoids.

When sarcopenia cannot be effectively treated with a correct diet only, food supplements, such as for example calcium, vitamin D and vitamin B12, branchedchain amino acids, precursors or metabolites thereof, are often introduced in the diet.

### Objects of the Invention

It is an object of the present invention to provide a new combination of active ingredients, particularly useful, but without limitation, in the treatment and/or prevention of sarcopenia and in general in the treatment of any condition or disease causing deterioration of the muscle mass and/or as anabolic steroid.

It is a further object of the present invention to provide a pharmaceutical and/or nutraceutical composition comprising the combination of the invention.

It is a further object of the invention to provide a composition for use in a method for the treatment and/or prevention of sarcopenia and in general for the treatment of any condition or disease causing deterioration of the muscle mass, comprising the administration of the combination or composition of the invention.

### Description of the Invention

Subject-matter of the invention, according to one of the aspects thereof, is a new combination of active ingredients comprising 3-hydroxy-3-methylbutanoic acid or a pharmacologically and pharmaceutically acceptable salt thereof, preferably calcium salt, a grape seed extract and a withania extract.

According to a preferred embodiment, the combination further comprises ornithine alpha-ketoglutarate.

3-Hydroxy-3-methylbutanoic acid (also named as 3-hydroxy-3-methylbutyric acid, herein in the following also HMB) is a metabolite of leucine amino acid. Its anabolic and anti-catabolic role is known. According to the present invention, 3-hydroxy-3-methylbutanoic acid is preferably used in the form of a pharmaceutically acceptable salt thereof, advantageously in the form of its calcium salt.

According to the present invention, grape seed extract (also named grapestones) means a leucocyanidin extract obtained from *Vitis vinifera L.* seeds, e.g. by water and acetone extraction. Such extract is commercially available and is for example marketed by Indena® Company. In particular, the extract marketed by Indena® Company comprises, in addition to leucocyanidins, soybean phospholipids as well.

According to the present invention, withania extract means preferably an extract of *Withania somnifera.* Preferably the extract is an extract from the roots of *Withania somnifera,* also named "Aswagandha dry extract". Also this product is commercially available and is for example marketed by Vivatis Pharma Company.

Ornithine alpha-ketoglutarate (herein in the following also OKG) is a known compound, generally used in the sports field as an anabolic steroid.

According to a preferred embodiment, the combination of the invention is a fixed combination constituted by 3-hydroxy-3-methylbutanoic acid or a pharmaceutically acceptable salt thereof/grape seed extract/withania extract/ornithine alpha-ketoglutarate, at a reciprocal weight ratio of 1/0.1-0.30/0.1-0.5/2-5 respectively.

According to a preferred embodiment, the combination of the invention is a fixed combination constituted by calcium 3-hydroxy-3-methylbutanoate/grape seed extract/withania extract/ornithine alpha-ketoglutarate, at a reciprocal weight ratio of 1/0.1-0.30/0.1-0.5/2-5 respectively, preferably about 1/0.15/0.30/3, advantageously about 1/0.16/0.16/2.3.

It has been now found surprisingly that the combination described above is particularly useful in the treatment of sarcopenia and, in general, in the treatment of any condition or disease causing deterioration of the muscle mass and also as an anabolic steroid. In particular, it has been found that the grape seed and withania extracts contribute to enhance the anabolic effect of 3-hydroxy-3-methylbutanoic acid and ornithine alpha-ketoglutarate.

According to the present invention, the term "sarcopenia" denotes herein any muscle mass loss, independently from its origin and includes strength and physical performance loss due to muscle catabolism.

The invention is for example useful to increase the muscle mass during training or in elderly and debilitated people and in people who have lost musculature due to inactivity or bedriddening; in the cachexia conditions; to protect the muscle from damages induced by an intense workout and to contrast fatigue feeling and/or to improve physical performances.

Subject-matter of the invention, according to another of the aspects thereof, is a pharmaceutical or nutraceutical composition comprising a combination of 3-hydroxy-3-methylbutanoic acid or a pharmaceutically acceptable salt thereof, preferably the calcium salt, a grape seed extract, a withania extract and optionally ornithine alpha-ketoglutarate, together with conventional vehicles and/or excipients.

According to a preferred embodiment, subject-matter of the invention is a pharmaceutical or nutraceutical composition comprising a combination of 3-hydroxy-3-methylbutanoic acid or a pharmaceutically acceptable salt thereof, preferably the calcium salt, a grape seed extract, a withania extract and ornithine alpha-ketoglutarate, together with conventional vehicles and/or excipients.

Preferably, the composition of the invention is for the oral use, but other administration routes can be effectively used.

According to an embodiment, object of the invention is a compositions comprising
- 1 to 4 g, preferably 1 to 3 g of 3-hydroxy-3-methylbutanoic acid or a pharmaceutically acceptable salt thereof;
- 100 to 500 mg, preferably 200 to 400 mg of grape seed extract, preferably as defined above;
- 100 to 1000 mg, preferably 200 to 800 mg of withania extract, advantageously withania somnifera; and
- 1 to 10 g, preferably 2 to 8 g of ornithine alpha-ketoglutarate,
together with conventional vehicles and/or excipients.

According to a preferred embodiment, object of the invention is a composition comprising
- 1 to 4 g, preferably 1 to 3 g of calcium 3-hydroxy-3-methylbutanoate;
- 100 to 500 mg, preferably 200 to 400 mg of grape seed extract, preferably as defined above;
- 100 to 1000 mg, preferably 200 to 800 mg of withania extract, advantageously withania somnifera; and
- 1 to 10 g, preferably 2 to 8 g of ornithine alpha-ketoglutarate,
together with conventional vehicles and/or excipients.

According to a preferred embodiment, object of the invention is a composition comprising
- about 1 g of calcium 3-hydroxy-3-methylbutanoate;
- about 150 mg of grape seed extract, preferably as defined above;
- about 300 mg of withania extract, advantageously withania somnifera; and
- about 3 g of ornithine alpha-ketoglutarate;
together with conventional vehicles and/or excipients.

According to a preferred embodiment, subject-matter of the invention is a composition comprising
- about 1.5 g of calcium 3-hydroxy-3-methylbutanoate;
- about 250 mg of grape seed extract, preferably as defined above;
- about 250 mg of withania extract, advantageously withania somnifera; and
- about 3.5 g of ornithine alpha-ketoglutarate;
together with conventional vehicles and/or excipients.

Such compositions are suitable for the oral administration to the subject to be treated, one or more times a day.

The dosage of the composition to be administered depends on the need, e.g. on the age of the subject, on the kind of disease, the health condition, etc. such dosages can be determined by the physician.

According to a preferred embodiment, the compositions are administered two times a day, for a total daily dosage of
- about 2-3 g of calcium 3-hydroxy-3-methylbutanoate;
- about 300-500 mg of grape seed extract, preferably as defined above;
- about 500-600 mg of withania extract, advantageously withania somnifera; and
- about 6-7 g of ornithine alpha-ketoglutarate.

However other dosages can be administered according to the need, as mentioned.

The subject to be treated with the composition of the invention is preferably a mammalian, in particular the human being.

The composition of the invention is preferably a pharmaceutical composition formulated for the oral use, e.g. in form of pharmaceutical or nutraceutical compositions such as tablets, optionally coated, granules, fine granules, powders, hard capsules, soft capsules, syrups, emulsions, suspensions and solutions suitable for the oral administration. However other pharmaceutical forms can be used.

According to a preferred embodiment, the composition of the invention is in solid or semi-solid oral form, advantageously selected among tablets, powders, granules and gel. Such composition can be taken alone or with water, or mixed with other foods, e.g. with yogurt or the like.

Alternatively, the composition of the invention can be in a ready-to-use drinkable form, such as for example in the form of sachets to drink, of the "stick pack" type, preferably in the gel form.

The kinds of pharmaceutical additives used for the preparation of the composition of the invention, the ratios of the amounts of the additives relative to the active ingredient and the methods to prepare the pharmaceutical composition can be suitably selected by the one skilled in the field. Organic or inorganic substances, or solid or liquid substances can be used as excipients and vehicles, provided that they are edible and pharmaceutically acceptable.

Examples of the excipients used for the preparation of the solid pharmaceutical compositions include for example maltodextrins, cellulose derivatives such as hydroxypropyl methyl cellulose, lactose, sucrose, sucrose esters, citric acid, silicon dioxide, amid, talc, cellulose, dextrins, kaolin, calcium carbonate, stearic acid or magnesium stearate, lactose, polyethylene glycol, mannitol, sorbitol, chelating agents, anti-caking agents, sugar substitutes, preservative and flavoring agents.

For the preparation of liquid compositions for the oral administration, a conventional inert diluent such as water or an oil, e.g. a vegetable oil, can be used. The liquid composition can include adjuvants such as wetting agents, suspending agent, sweeteners, flavorings, colorings and preservatives in addition to the inert diluent. The liquid composition can be included in capsules of an absorbable material, such as gelatin.

The sugar substitutes can be one or more natural sugars, optionally reduced, such as for example sucrose, sucralose, dextrose, xylitol, mannitol or sorbitol, or a synthetic product such as for example sodium saccharin, aspartame, acesulfame K or sucralose. Acidifying agents can also be added.

Flavoring agents are pharmaceutically acceptable flavors and tastes from synthetic oils or natural oils, the latter extracted from plants, flowers, fruits and combinations thereof, such as for example cinnamon, mint, anise, and citrus fruits leaves, bitter almonds, citrus fruits, in particular orange and/or lemon oils, lime, vanilla, chocolate and grapefruit. Advantageously chocolate, vanilla or eucalyptus flavors and fruit essences, in particular apple, pear, peach, strawberry, apricot, orange, lemon and grapes, can be used.

The composition of the invention can further comprise other active ingredients or vegetable extracts useful in the treatment and/or the prevention of sarcopenia.

By way of example, the compositions of the invention can comprise amino acids, e.g. one or more amino acids selected from L-leucine, L-valine, L-isoleucine, L-histidine, L-lysine, L-methionine, L-cystine, L-cysteine, L-phenylalanine, L-tyrosine, L-threonine, L-glutamine, L-arginine and L-tryptophan and/or one or more vitamins, e.g. Vitamin B6 and Vitamin D3.

The dosages of the amino acids and vitamins can be easily identified by the recommended daily allowances or can be selected by the physician or the dietitian based on the needs of the subject to be treated. Examples are provided in the Experimental Section of the present invention.

By way of example about 5-20 g, e.g. 8-12 g, advantageously 10 g of an amino acid mixture and/or about 1-3 mg of group B Vitamins, preferably Vitamin B6 and/or about 10-30 micrograms of a group D vitamins, preferably Vitamin D3, can be added to the composition.

Other components can be further added to the composition of the invention, such as for example creatine, e.g. at an amount of 0.5-3 grams.

In addition to the treatment of sarcopenia, as defined above, the combination and the composition of the invention, preferably the composition comprising amino acids and/or vitamins, can be conveniently used as anabolic agent, in particular by sportsmen or in any case by subjects who aims at increasing the muscle mass.

Preferred additional components are indicated in the illustrative examples of the compositions of the invention described in the Experimental Section.

The composition of the invention can be packaged in unit doses or multi-dose packagings, according to conventional methods well known by the one skilled in the field.

According to a preferred embodiment, the compositions of the invention are packaged in oral unit doses, e.g. sachets, capsules or tablets.

Subject-matter of the invention, according to another of the aspects thereof, is the use of the pharmaceutical or nutraceutical combination and composition according to the invention for the treatment and/or prevention of sarcopenia.

Therefore the combination and the compositions of the invention are particularly useful to increase the muscle mass (by increasing the protein synthesis and reducing the muscle deterioration) during training or in elderly and debilitated people and in people who have lost musculature due to inactivity or bedriddening; to protect the muscle from the damages induced by an intense workout; and to contrast fatigue feeling and/or to improve physical performances.

The combination and the composition according to the invention for their use in the treatment of sarcopenia and for the uses set forth above are a further aspect of the invention.

Subject-matter of the invention, according to another of the aspect thereof, is a method for the treatment and/or the prevention of sarcopenia, comprising the administration of an effective dose of the combination and/or the composition according to the invention to a subject in need thereof.

The combination of the invention has been assayed in various *in vivo, in vitro* and *ex-vivo* experiments.

### Experiment 1

### Test in rat following a chronic treatment in an in vivo model of sarcopenia

A model of muscle damage has been reproduced by an intraperitoneal administration to the rat of a daily dose of 600 microg/kg of dexamethasone for 15 days until an evident loss of body weight with respect to the control group treated with the vehicle.

The combination of the substances under examination has been administered orally each day for the whole duration of the treatment. The efficiency of the so composed combination has been assessed:
- 250 microg/kg grape seed extract
- 250 microg/kg withania extract
- 300 microg/kg HMB
- 500 microg/kg OKG.

Starting from day 1, the different groups of animals have been treated respectively with:
- dexamethasone alone,
- dexamethasone and the combination set forth above,
- the respective vehicles (controls).

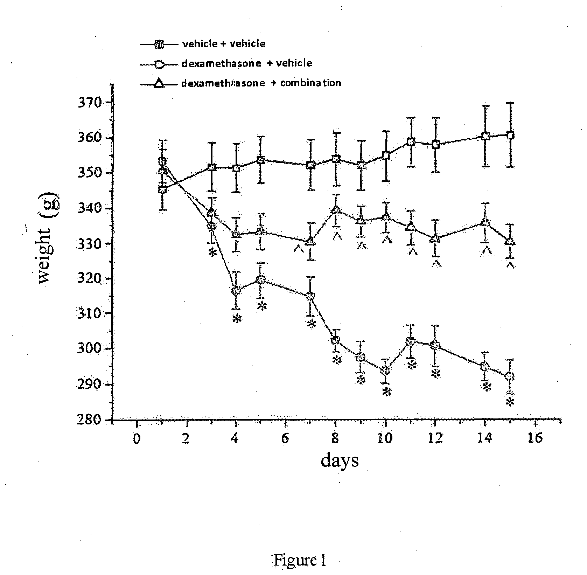

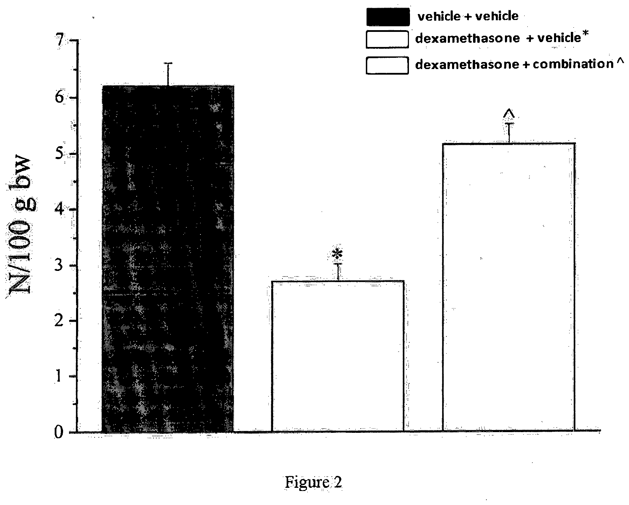

The body weight has been monitored daily as reported in Figure 1. The treatment with dexamethasone induces a sudden loss of weight which goes on progressively until the end of the observation at day 15. The animals receiving the treatment with the combination have a weight which is always higher than that of those treated with dexamethasone alone. Starting from day 7, such increase is significant and the weight remains constant until day 15. The body weight increase is correlated with an improvement of the functional abilities evaluated by means of Grip test which allows the determination of the strength applied by the animal to keep the grip and counter the pull. As it is shown in Figure 2 the effect of strength reduction induced by dexamethasone is significantly reduced by the treatment with the combination.

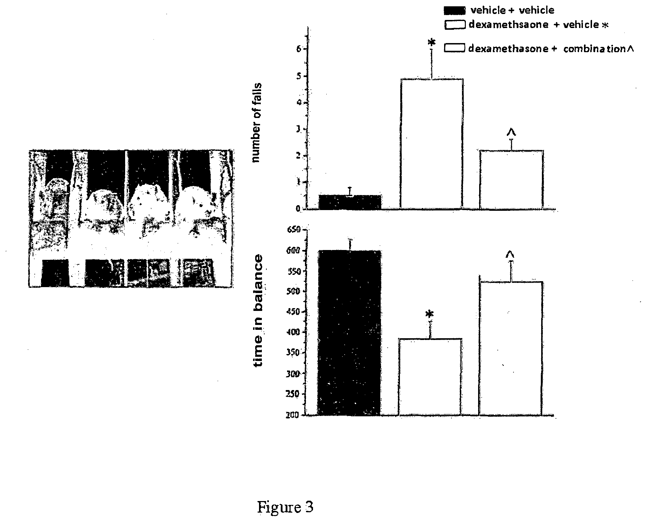

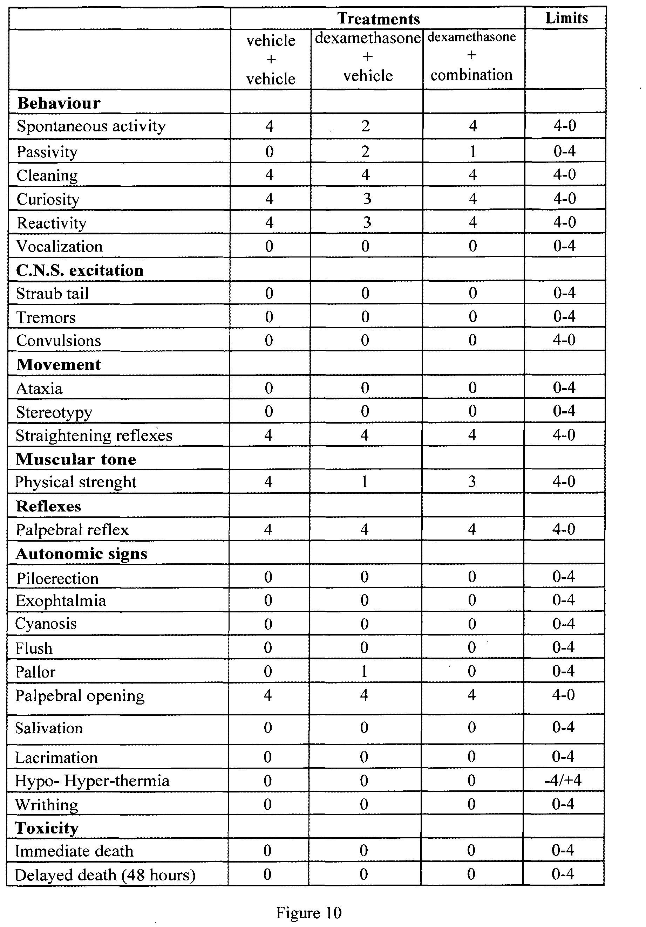

The higher strength corresponds to a better motor coordination stimulated in the "Rota rod test" by the requirement of keeping balance on a rotating rod. The animals treated frequently with the combination show a reduction of the number of falls and a longer period of riding time in balance on the semi-rotating rod (Figure 3). In order to extend such investigation, the animals underwent an evaluation of their autonomic, neurological and motor abilities by observing a series of parameters reported in Table 1 (Figure 10). Such evaluations, summarized as Irwin test, allowed to highlight the protective abilities of the combination against the alterations induced by dexamethasone and, not less important, allowed to exclude damages of the neurological profile induced by the combination thus confirming an excellent safety profile. During the test, the evaluation of the motor and neurological abilities has been carried out by Irwin test performed at day 15. The signs have been evaluated in a semi-quantitative way according to a predetermined scale (0 to+ 4,-4 to 0, 0-4 to + 4). The data refer to an evaluation of 10 rats per group divided into two different experimental sessions, *P<0.01 vs vehicle + vehicle; 11 P<0.01 vs dexamethasone + vehicle.

At the end of the behavioral observations (day 15), the animals have been sacrificed and some peculiar muscles of the back paw have been explanted and analyzed.

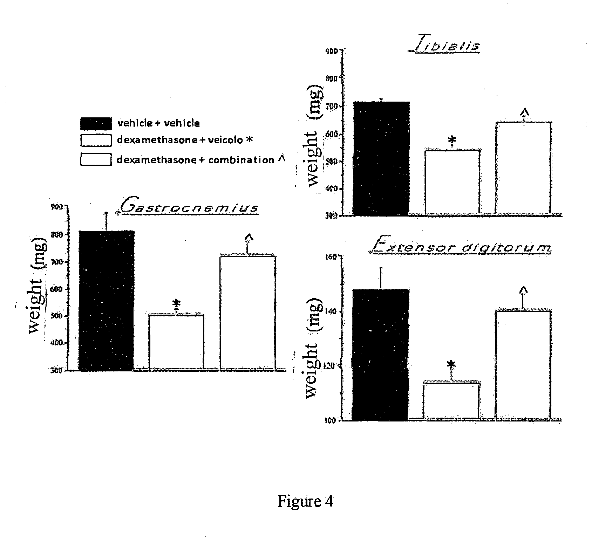

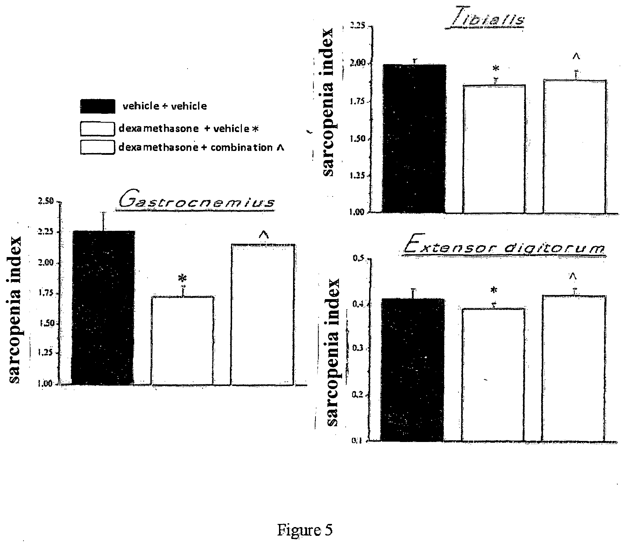

Figure 4 shows the dramatic weight reduction of the gastrocnemius, tibialis and extensor digitorum muscles induced by dexamethasone. It should be noted that the body weight reduction of the animals is about 20% following a treatment with dexamethasone whereas the muscle weight decreases of 40%, indicating a higher damage of the muscle structures with respect to other tissues. The same muscles collected from the animals treated with the combination have a significantly higher weight. The ability of the combination to almost completely prevent such alteration suggests that the tonic properties of the tested combination are almost completely directed towards the muscle tissue. However it is reported that only for the gastrocnemius muscle there is an appreciable improvement of the sarcopenia index (Figure 5). Such value is obtained by dividing the muscle weight by the total body weight, since the effects of the combination result in an overall increase of the body weight (clearly correlated to an improved wellbeing of the animal), the differences of such ratio result to be levelled by the simultaneous increase of the numerator and the denominator.

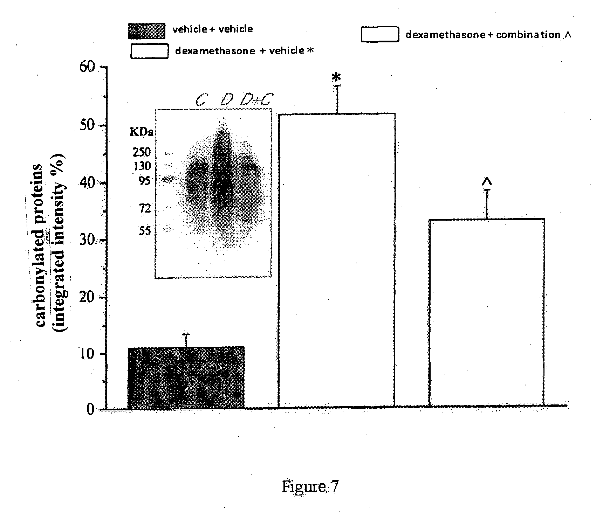

In order to investigate a possible mechanism for the efficiency shown by the combination, the oxidation state of the muscle has been evaluated by measuring the oxidation of lipids and proteins. The gastrocnemius muscle has been processed and reacted with thiobarbituric acid in order to allow the detection of the reactive substances of the acid (Thiobarbituric acid reactive substances-TBARS; Figure 5). Dexamethasone promotes a twofold increase of lipid peroxidation relative to the controls treated with the vehicle. Even more striking is the oxidation effect of the proteins evaluated as the presence of carbonylated proteins which increase of about 5 times (Figure 7). In both cases the treatment with the combination significantly prevents the oxidative stress of both lipids and proteins.

### Experiment 2

### Test in rat following a chronic treatment in an in vitro model of sarcopenia.

The study of the trophic action of the muscle of the investigated compounds has been carried out in a C2C12 murine myoblast line. The alterations have been produced by means of incubations for increasing times with 1µM dexamethasone (Lu et al. Int jour Biol Mol 2013, 61:7-16) which can induce muscle atrophy; the so-activated glucocorticoid receptor increases the transcription of its target genes which promote the expression of the proteins belonging to the FoxO family and of E3 ubiquitin ligases (Shimizu et al., Cell Metab, 13:170-182, 2011) acting at muscle cell level reducing the trophism and new formation. The action of the combination under examination has been highlighted following incubation with scalar concentrations of the single compounds or in mixture, in particular withania vegetable extracts, red grape seeds and calcium salt of hydroxymethylbutyrate have been tested.

The treatment of myoblasts with dexamethasone 1 microM for 48 h significantly reduces the cell viability evaluated by the MTT assay (see the Experimental Section) by about 70%.

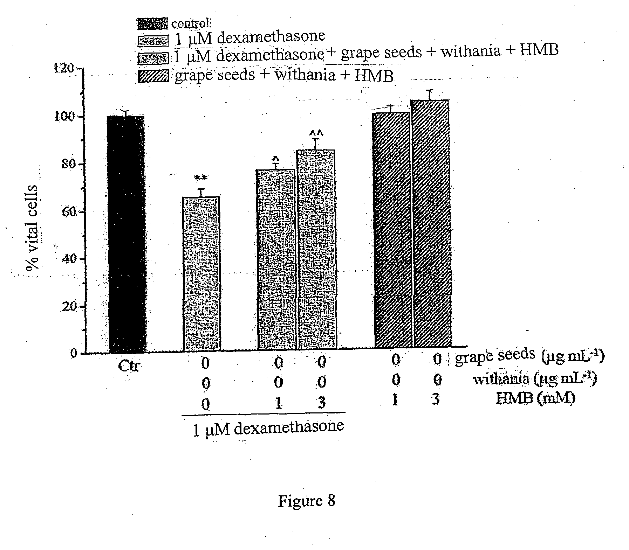

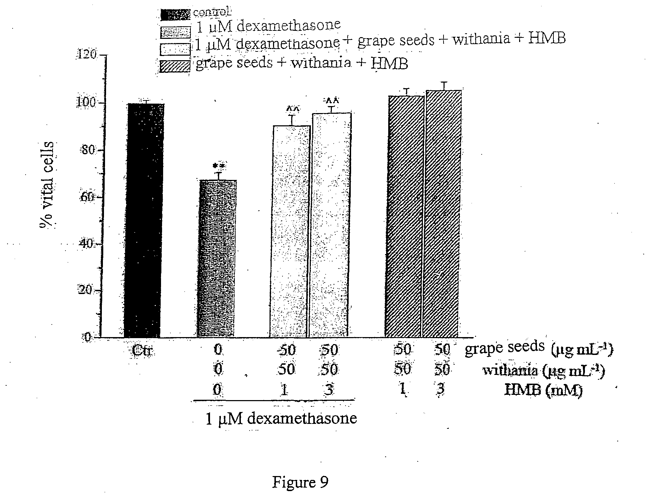

Figures 8 and 9 clearly show how the addition of grape seed and withania extracts significantly increase the HMB activity on the cell protection, following the cell damage produced by dexamethasone.

### Experiment 3

### Test in rat following a chronic treatment in an ex vivo model of sarcopenia.

The effect of the daily oral administration has been evaluated on a model of muscle damage induced by dexamethasone (administered intraperitoneally to the rat at the 600 µg kg dose--daily for 15 days; Yamamoto et al., Muscle and Nerve, 41:819--827, 2010)

During the treatment the body weight and the strength applied have been monitored by the Grip test, the motor coordination and the resistance to movement by Rotarod test, the neurological picture by EON test. The functional improvements induced by the mixture under examination have been confirmed, as highlighted in the previous report.

At the end of the treatment, morphometric and molecular measurements have been carried out on the single muscles, in particular the gastrocnemius and extensor digitorum muscles have been evaluated.

Histological analysis carried out by means of hematoxylin---eosin (Figure 11) and the phalloidin labelling of myosin (Figure 12) showed a significant alteration of the structure in the extensor digitorum muscle following the treatment with dexamethasone. The treatment with the mixture ("complex" in the figures) induced an improvement which is quantifiable by the morphometric analysis. In particular the complex can reactivate the area of the fibers (Figure 13), the number of fibers per section (Figure 14), the perimeter of the fibers (Figure 15) and, finally, the minimal Feret's diameter considered as the most correct measure for the evaluation of the morphological state of the muscle. The Feret's diameter is defined as the minimal distance between two fiber tangents parallel each other; it allows to evaluate the real measure of fibers that are non-homogeneous by their nature and preparation (Figure 16). In Table 1 the lipofuscin level measured in the muscle tissue is reported as a stress marker of the autophagosome-lysosome processes. The treatment with the complex under examination significantly reduces the amount of lipofuscin. Focusing on myotube states, Figure 17 depicts the normalization of the diameter in the muscles explanted from animals repeatedly treated with the complex. No alterations concerning the number of nuclei per each myotube have been found. The analysis of the gastrocnemius muscle showed analogous results.

### Experiment 4

### Test in rat following a chronic treatment in an in vivo model of sarcopenia

It has been evaluated on a model of muscle damage induced by chemotherapy treatment with the antitumor agent oxalyl platinum.

In particular, the muscle damage model has been reproduced by intraperitoneally administering to the rat oxalyl platinum at the daily dose of 2.4 mg kg-1 for 15 days (Cavaletti et al., Eur J Cancer 2001; Di Cesare Mannelli et al., Exp Neurol 2014) until a clear loss of body weight with respect to the control group treated with the vehicle.

The combination of the substances under examination has been administered orally each day for the whole duration of the chemotherapy treatment. The efficiency of the so composed combination has been determined:
- 250 mg kg-1 grape seed extract (Vitis vinifera)
- 250 mg kg-1 withania extract (Withania somnifera)
- 300 mg kg-1 HMB
- 500 mg kg-1 OKG

Starting from day 1 the different groups of animals have been treated respectively with
- oxalyl platinum,
- oxalyl platinum and the combination,
- the respective vehicles.

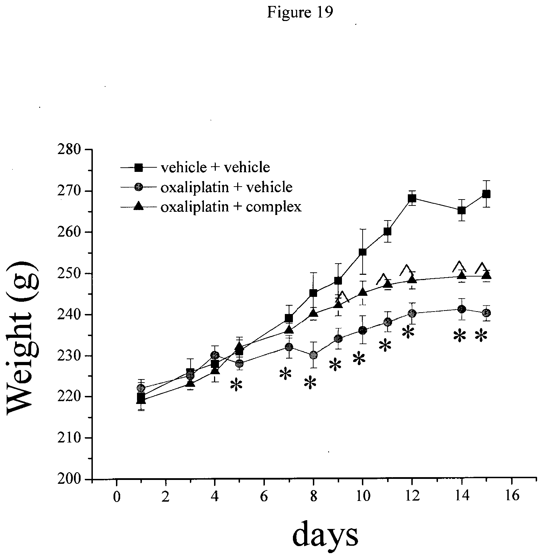

The body weight has been monitored daily as reported in Figure 19. The treatment with oxalyl platinum prevents the physiological weight gain during time. Starting from day 9 the animals receiving the treatment with the mixture show a weight which is significantly higher than that of those treated with oxalyl platinum alone.

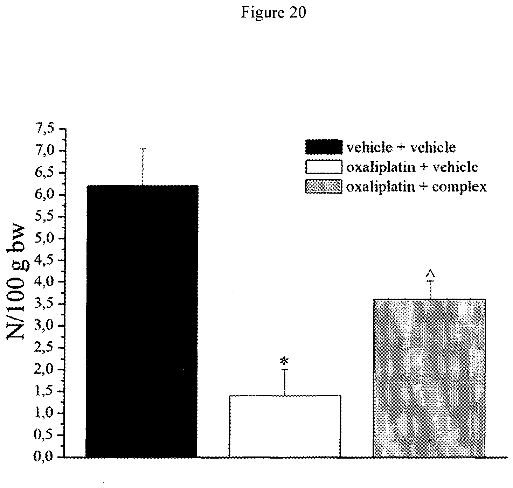

The body weight increase is correlated with an improvement of the functional abilities evaluated by means of Grip test which allows the determination of the strength applied by the animal to keep the grip and counter the pull. As it is shown in Figure 20 the effect of strength reduction induced by oxalyl platinum is significantly prevented by the treatment with the complex.

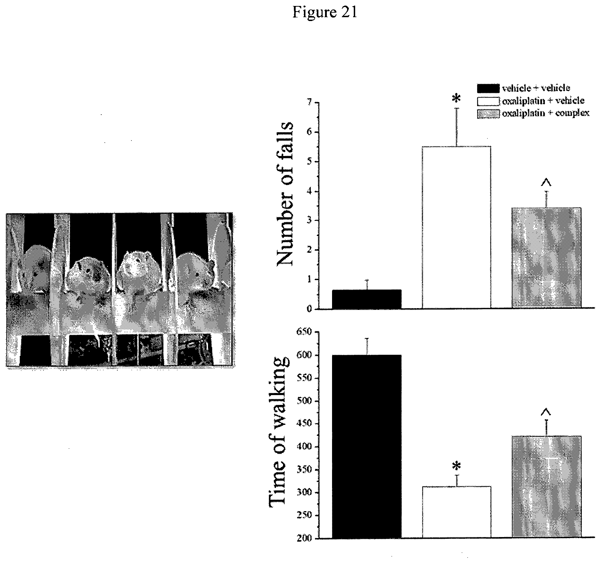

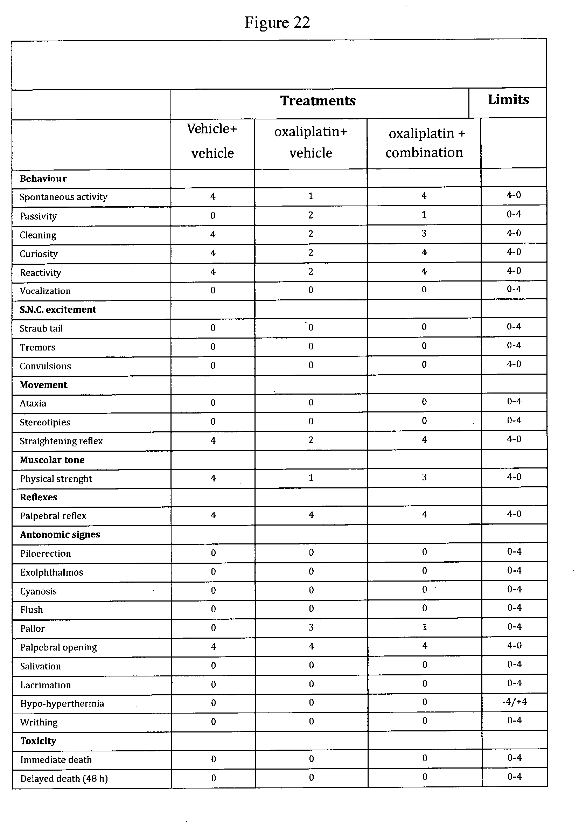

The higher strength corresponds to a better motor coordination stimulated in the Rota rod test by the requirement of keeping balance on a rotating rod. The animals treated repeatedly with the molecular complex constituted by red grape and withania extracts, HMB and OKG have a reduction of the number of falls and a longer time of residence in balance during movement on the semi-moving rod, thus suggesting higher resistance to the physical activity (Figure 21). In order to extend such investigation the animals underwent an evaluation of their autonomic, neurological and motor abilities by observing a series of parameters reported in the Table of Figure 22. Such evaluations, summarized as Irwin test, allowed to highlight the protective abilities of the complex against the alterations induced by the chemotherapeutic agent and, not less important, allowed excluding damages of the neurological profile induced by the mixture thus confirming a good safety profile.

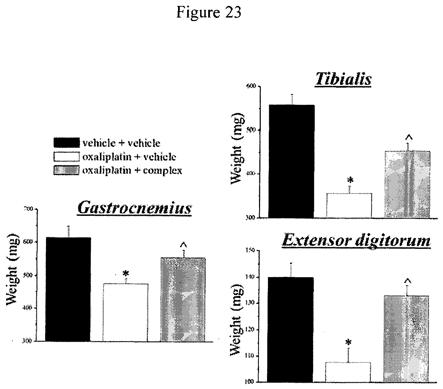

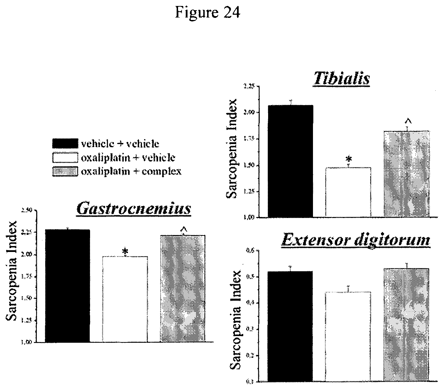

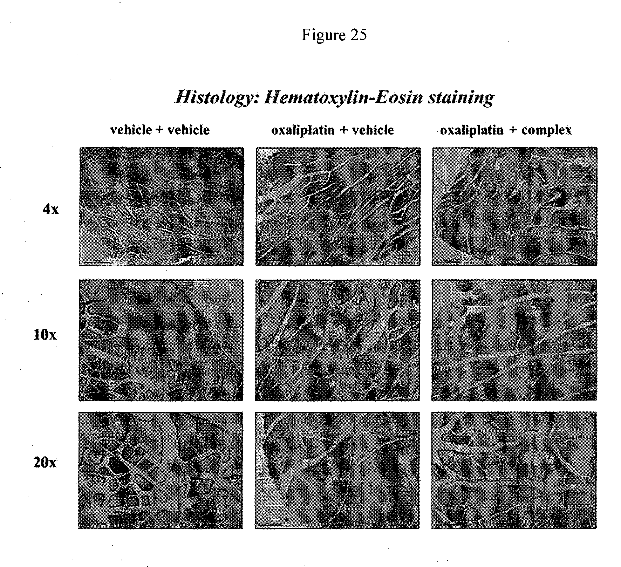

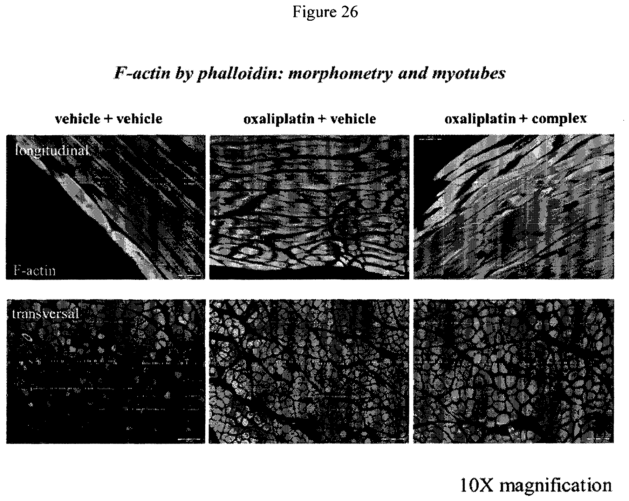

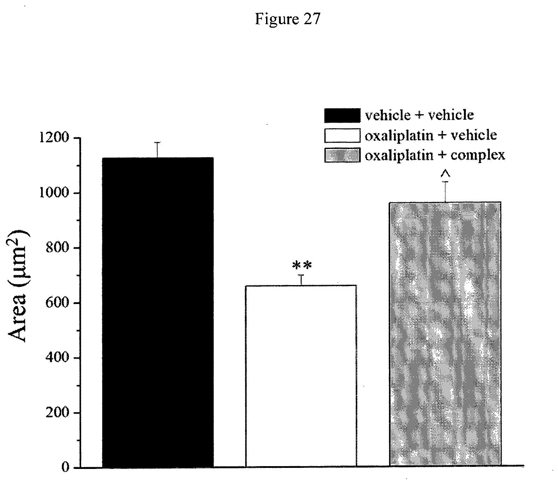

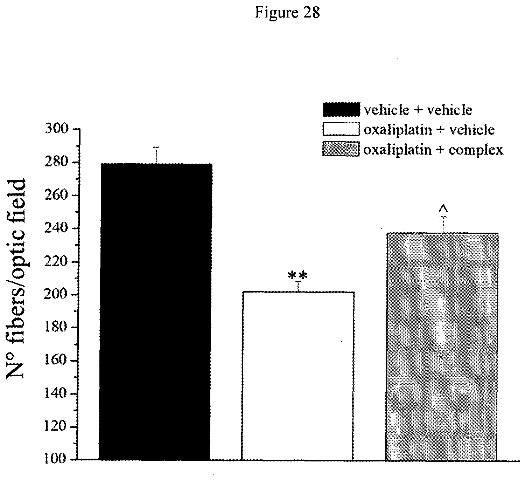

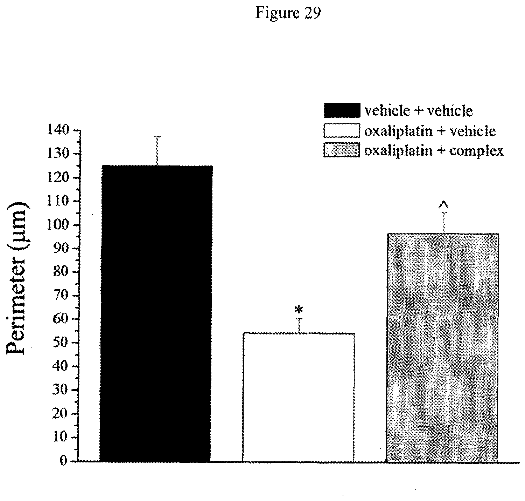

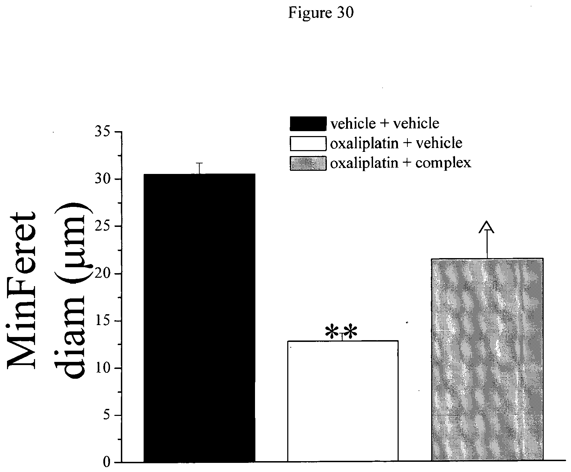

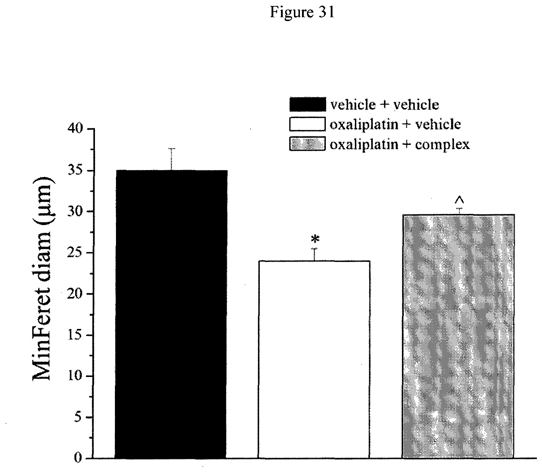

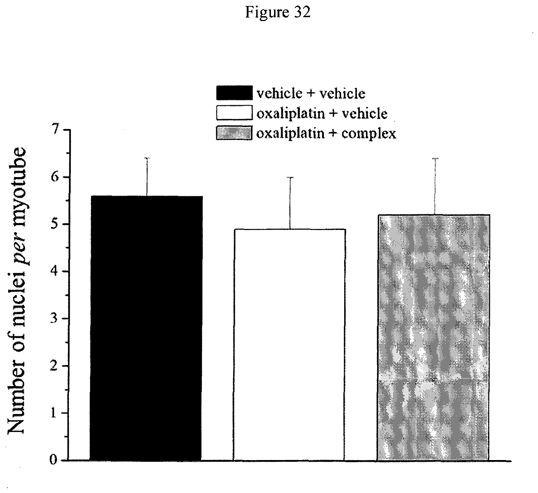

At the end of the behavioral observations (day 15), the animals have been sacrificed and some muscles of the back paw have been explanted and analyzed. Figure 23 shows the dramatic weight reduction of the gastrocnemius, tibialis and extensor digitorum muscles induced by oxalyl platinum. It should be noted that the body weight reduction of the animals is about 15% following a treatment with dexamethasone whereas the muscle weight decreases by 50% indicating a higher damage of the muscle structures with respect to other tissues. The same muscles collected from the animals treated with the complex have a significantly higher weight. It is reported that for the gastrocnemius and tibialis muscles there is an appreciable improvement of the sarcopenia index (Figure 24). In addition extensor digitorum and gastrocnemius muscles underwent a morphometric and molecular study. Histological analysis carried out by means of hematoxylin-eosin (Figure 25) and the phalloidin labelling of myosin (Figure 26) showed a significant alteration of the structure in the extensor digitorum muscle following the treatment with oxalyl platinum. The treatment with the mixture ("complex" in the figures) induced an improvement which is quantifiable by the morphometric analysis. In particular the complex can reactivate the area of the fibers (Figure 27), the number of fibers per section (Figure 28), the perimeter of the fibers (Figure 29) and, finally, the minimal Feret's diameter (Figure 30) considered as the most correct measure for the evaluation of the morphological state of the muscle. The Feret's diameter is defined as the minimal distance between two fiber tangents parallel each other; it allows to evaluate the real measure of fibers that are non-homogeneous by their nature and preparation. Focusing on myotube states, Figure 31 depicts the normalization of the diameter in the muscles explanted from animals repeatedly treated with the complex. No alterations concerning the number of nuclei per each myotube have been found (Figure 32). The analysis of the gastrocnemius muscle showed analogous results.

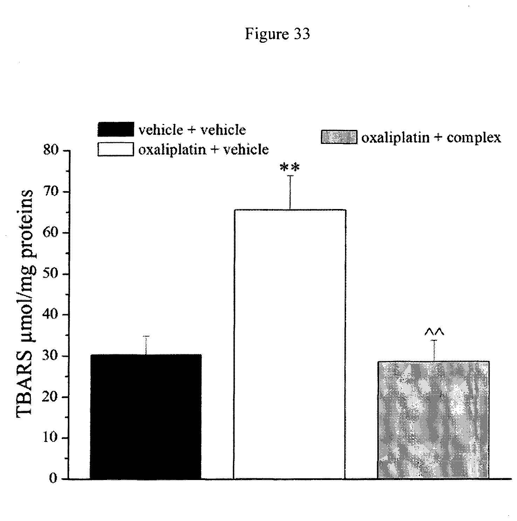

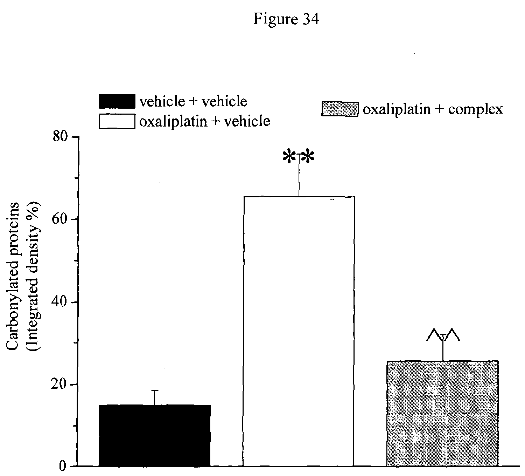

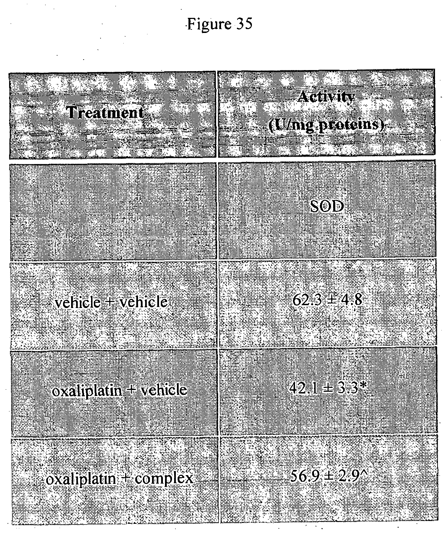

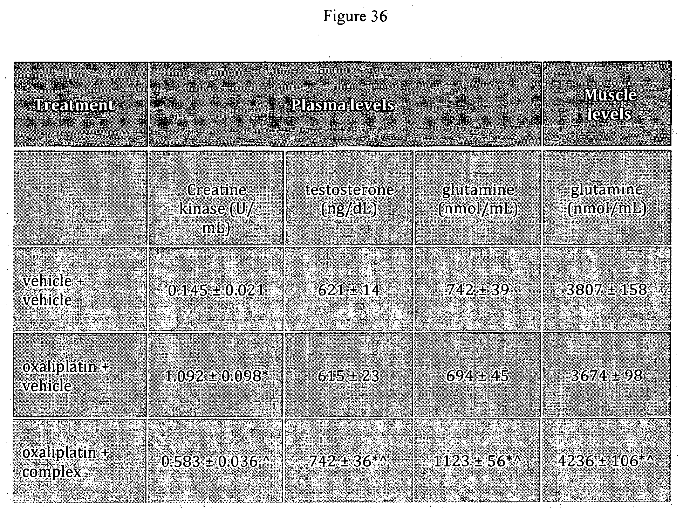

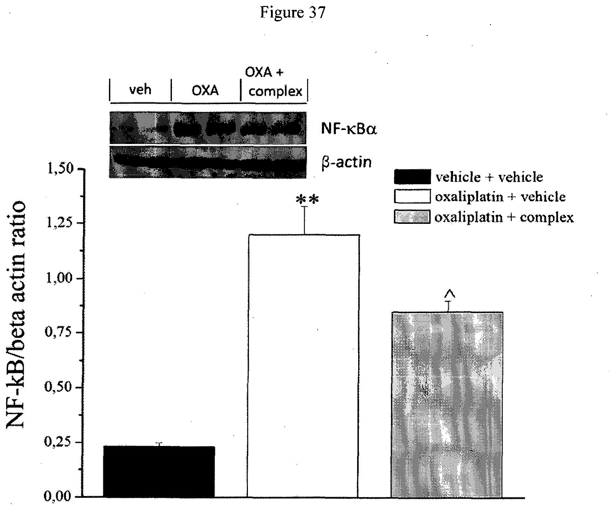

In order to investigate a possible mechanism for the efficiency shown by the complex, the oxidation state of the muscle has been evaluated by measuring the oxidation of lipids and proteins. The extensor digitalis muscle has been processed and reacted with thiobarbituric acid in order to allow the detection of the reactive substances of the acid (TBARS; Figure 33). In the animals treated with oxalyl platinum alone a twofold increase of lipid peroxidation relative to the controls treated with the vehicle has been observed. Even more striking is the oxidation effect of the proteins evaluated as the presence of carbonylated proteins which increase of about 4 times (Figure 34). In both cases the treatment with the complex significantly prevents the oxidative stress of both lipids and proteins. Redox dysregulation induced by oxalyl platinum has been also verified by measuring the superoxide dismutase (SOD) activity in the muscle (Figure 35). The reduction of the enzyme activity induced by oxalyl platinum is significantly prevented by the treatment with the complex. The antioxidant protection picture is in agreement with the pharmacodynamic profile of the grape seed extract. Moreover, the recovery of the muscle tissue has been evaluated by measuring the creatine kinase plasma levels (Figure 36), a muscle enzyme released in the bloodstream following a muscle damage. The dramatic increase (about 7 times) induced by oxalyl platinum is reduced by 50% by the treatment with the mixture. Such effect can be related to the properties of the withania extract. Analogously, the plasma increase of testosterone induced by the complex is in agreement with what stated in the literature concerning withania effects. On the other hand the glutamine increase, favoring the protein synthesis and inhibiting the muscle degradation, is ascribable to OKG, an amino acid precursor. Finally, Figure 37 shows the increase, oxalyl platinum-dependent, of the NF-kB (nuclear factor kappa-light-chain-enhancer of activated B cells) expression in the muscle, a protein complex with catabolic activity. The repeated treatment with the complex prevents the increase of such inflammation mediator.

### Conclusions

The experimental results clearly demonstrate that the combination of the invention effectively prevents the muscle damage induced by dexamethasone. A general increase of body weight is supported by a pronounced promotion action of the muscle mass. Such effects can improve strength and motor abilities of the animals. The muscle protection is associated with a restoration of the redox balance of the muscle structure. Moreover, the results show that the components of the combination show a synergistic effect, since the simultaneous administration of HMB, grape seeds and withania extract produce an effect, with respect to the damages induced by dexamethasone, which is far higher than the one that could be achieved with the administration of HMB alone.

The combination can further counter the muscle damage induced by oxalyl platinum. A general increase of body weight is supported by a pronounced promotion action of the muscle mass. Such effects can improve strength and motor abilities of the animals. The muscle protection is combined with a restoration of the redox balance of the muscle structure together with an increase of the anabolic activities and a decrease of the catabolic signals.

The materials and the methods used are reported in the following Experimental Section.

### Brief Description of the Drawings

Figure 1. Evaluation of the body weight as a function of time. Data are reported as mean ± S.E.M. of 10 rats per group, which are divided into two different experimental sessions, *P<0.01 vs vehicle + vehicle; ^p<0.01 vs dexamethasone + vehicle.
Figure 2. Evaluation of grip force. Grip strength meter test performed at day 15. Data are reported as mean ± S.E.M. of 10 rats for each group divided into two different experimental sessions, *p<0.01 vs vehicle + vehicle; ^p<0.01 vs dexamethasone + vehicle.
Figure 3. Evaluation of motor coordination. Rota rod test performed at day 15, the number of falls and the time spent in balance on the rotating rod at 600 s have been evaluated. Data are reported as mean ± S.E.M. of 10 rats per group, which are divided into two different experimental sessions, *p<0.01 vs vehicle + vehicle; ^p<0.01 vs dexamethasone + vehicle.
Figure 4. Ex-vivo evaluation of muscle weight. At day 15 some muscles from the back paw
   have been explanted and weighted. In particular the weights of the gastrocnemius, tibialis and extensor digitorum muscles are reported. Data are reported as mean ± S.E.M. of the single muscles obtained by 10 rats per group, which are divided into two different experimental sessions, *p<0.01 vs vehicle + vehicle; ^p<0.01 vs dexamethasone + vehicle.
Figure 5. Evaluation of the sarcopenia index referred to the weight of the back paw. At day 15 some muscles of the back paw have been explanted and weighted. In particular the weights of the gastrocnemius, tibialis and extensor digitorum muscles are reported. The sarcopenia index has been obtained by dividing the weight of each muscle by the total weight of the animal. Data are reported as mean ± S.E.M. of the values obtained by 10 rats per group, which are divided into two different experimental sessions, *p<0.01 vs vehicle + vehicle; ^p<0.01 vs dexamethasone + vehicle.

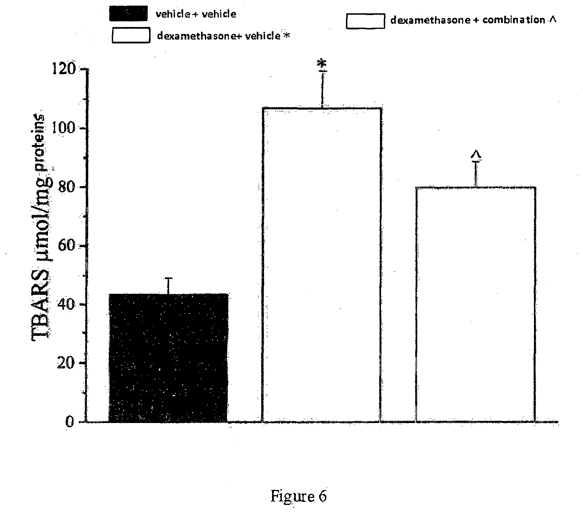

Figure 6. Evaluation of the muscle lipid peroxidation. At day 15 the gastrocnemius muscle has been explanted. The measurement of the oxidation state of the lipids has been carried out by means of the reaction with thiobarbituric acid and the results are expressed as TBARS amount (Thiobarbituric acid reactive substances.). Data are reported as mean ± S.E.M. of the values obtained by 10 rats per group, which are divided into two different experimental sessions, *p<0.01 vs vehicle + vehicle; ^p<0.01 vs dexamethasone + vehicle.
Figure 7. Evaluation of muscle protein carbonylation. At day 15 the gastrocnemius muscle has been explanted. The measurement of the oxidation state of the proteins has been carried out by means of western blot after reacting the protein homogenate with DNPH (2-4-dinitrophenylhydrazine). For each sample the normalization with respect to the expression of the beta-actin reference protein has been carried out. In the plot the measurement of the integrated density and in the insert a representative image of the blot with the respective molecular weights are shown, the control is denoted as C, the one treated with dexamethasone as D, the one treated with the combination D + C. Data are reported as mean ± S.E.M. of the values obtained by 10 rats per group, which are divided into two different experimental sessions, *p<0.01 vs vehicle + vehicle; ^p<0.01 vs dexamethasone + vehicle.
Figure 8: Evaluation of the cell viability on C2C12 (myocytes) treated with 1 µM dexamethasone with and without HMB. 48 hours treatments. MMT test (with 3-(4,5-dimethylthiazol-2-yl)-2,5-diphenyltetrazolium bromide). **p<0.01 vs dexamethasone; ^^p<0.01 vs 1 dexamethasone.
Figure 9: Evaluation of the cell viability on C2C12 (myocytes) treated with 1 µM dexamethasone with and without a mixture of grape seed, withania extracts and HMB. 48 hours treatments. MMT test (with 3-(4,5-dimethylthiazol-2-yl)-2,5-diphenyltetrazolium bromide). **p<0.01 vs dexamethasone; ^^p<0.01 vs 1 dexamethasone.
Figure 10 depicts the results of the Irwin test, obtained with the combination set forth in Experiment 1.

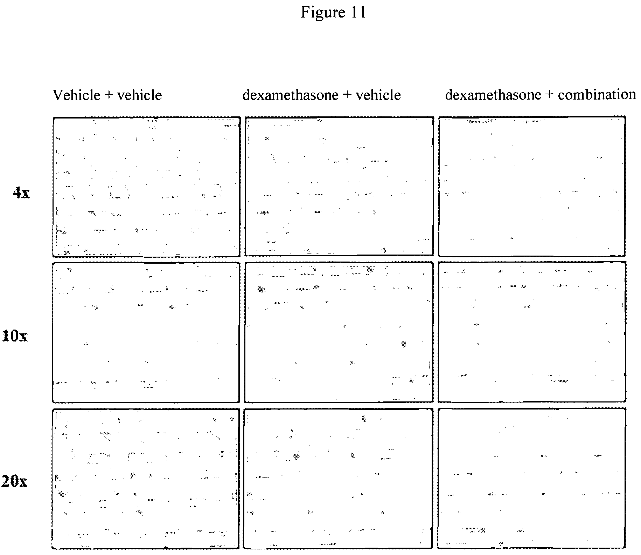

Figure 11 shows the histology of the muscle by staining with hematoxylin-eosin of Experiment 3.

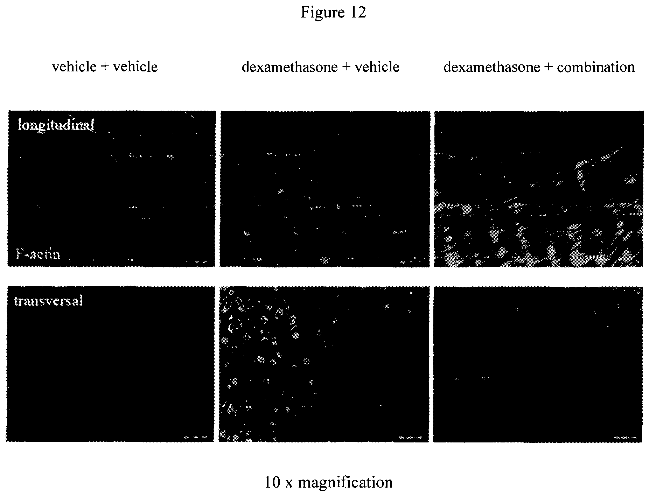

Figure 12 shows the immunohistochemistry of F-actin by phalloidin of Experiment 3.

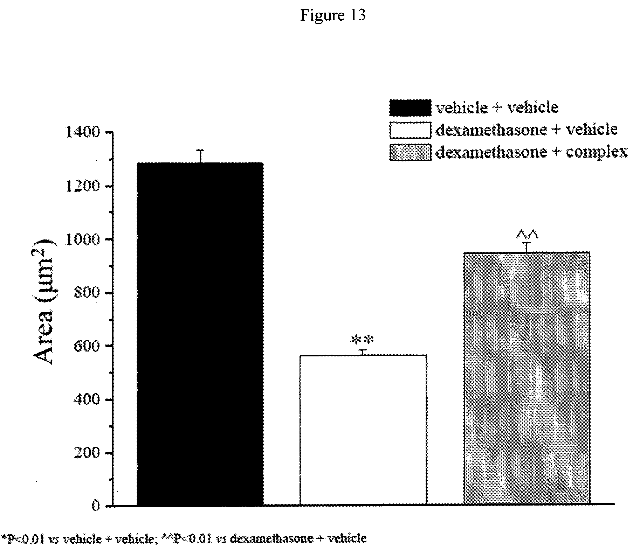

Figure 13 shows the area of the fibers of Experiment 3.

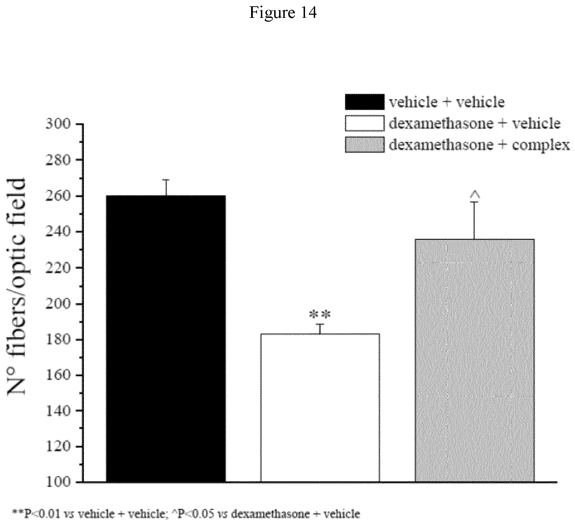

Figure 14 shows the number of the fibers of Experiment 3.

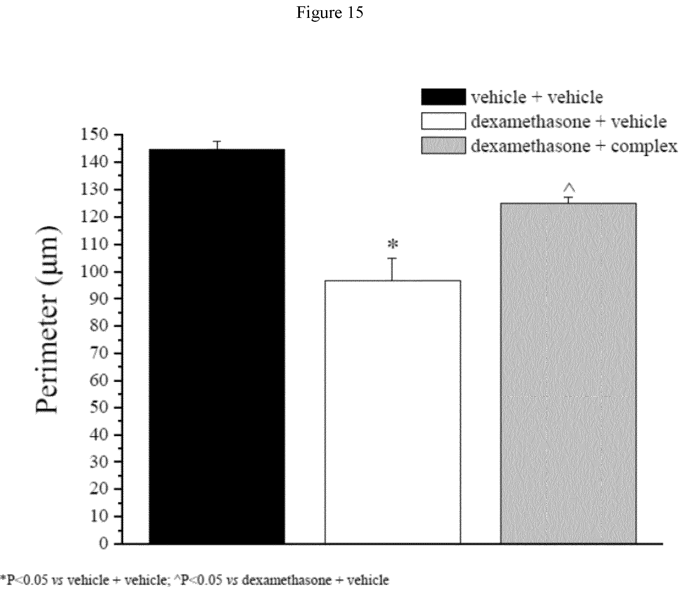

Figure 15 shows the perimeter of the fibers of Experiment 3.

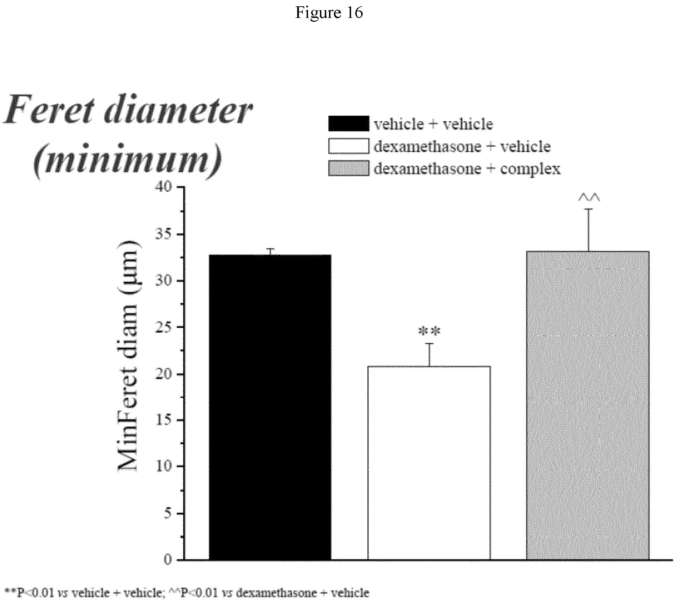

Figure 16 depicts the minimal Feret's diameter of Experiment 3.

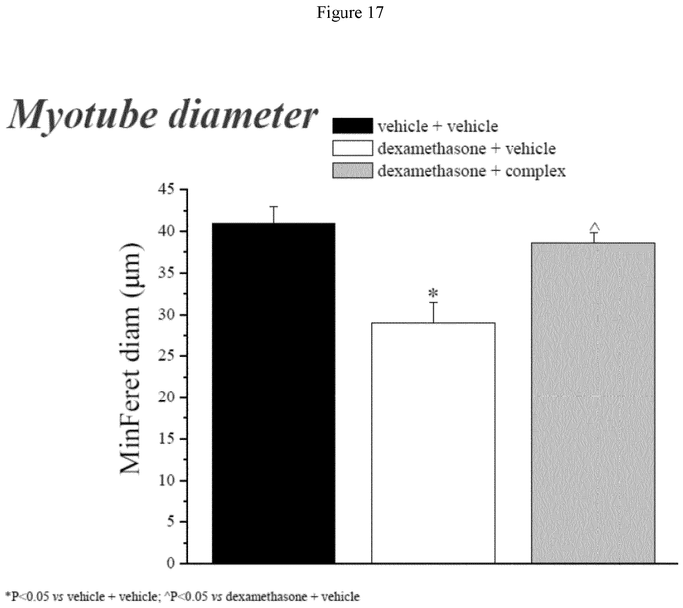

Figure 17 depicts the myotubes diameter of Experiment 3.
Figure 19 shows the evaluation of the body weight as a function of time. Data are reported as mean ± S.E.M. of 10 rats per group, which are divided into two different experimental sessions, *P<0.05 vs vehicle + vehicle; ^P<0.05 vs oxalyl platinum + vehicle.
Figure 20 shows the evaluation of the lost strength. Grip strength meter test performed at day 15. Data are reported as mean ± S.E.M. of 10 rats per group, which are divided into two different experimental sessions, *P<0.05 vs vehicle + vehicle; ^P<0.05 vs oxalyl platinum + vehicle.
Figure 21 shows the evaluation of the motor coordination and resistance to the exercise. Rota rod test performed at day 15, the number of falls and the time spent in balance on the rotating rod at 600 s have been evaluated. Data are reported as mean ± S.E.M. of 10 rats per group, which are divided into two different experimental sessions, *P<0.05 vs vehicle + vehicle; ^p<0.05 vs oxalyl platinum + vehicle.
Figure 22 shows the evaluation of the motor and neurological abilities by Irwin test carried out at day 15. The signs have been evaluated in a semi-quantitative way according to a predetermined scale (0 to + 4,-4 to 0, or -4 to + 4). Data refer to an evaluation of 10 rats per group, which are divided into two different experimental sessions.
Figure 23 shows the ex-vivo evaluation of the muscle weight. At day 15 some muscles of the back paw have been explanted and weighted. In particular the weights of the gastrocnemius, tibialis and extensor digitorum muscles are reported. Data are reported as mean ± S.E.M. of the single muscles obtained by 10 rats per group, which are divided into two different experimental sessions, *P<0.05 vs vehicle + vehicle; ^p<0.05 vs oxalyl platinum + vehicle.
Figure 24 shows the evaluation of the sarcopenia index referred to the weight of the back paw muscles. At day 15 some muscles of the back paw have been explanted and weighted. In particular the weights of the gastrocnemius, tibialis and extensor digitorum muscles are reported. The sarcopenia index has been obtained by dividing the weight of each muscle by the total weight of the animal. Data are reported as mean ± S.E.M. of the values obtained by 10 rats per group, which are divided into two different experimental sessions, *P<0.05 vs vehicle + vehicle; ^p<0.05 vs oxalyl platinum + vehicle.
Figure 25 shows a histological sample.
Figure 26 shows the morphometry and the myotubes.
Figure 27 shows the area of the fibers. Data are reported as mean ± S.E.M. of the values obtained by 10 rats per group, which are divided into two different experimental sessions,** P<0.01 vs vehicle + vehicle; ^p<0.05 vs oxalyl platinum + vehicle.
Figure 28 shows the number of the fibers. Data are reported as mean ± S.E.M. of the values obtained by 10 rats per group, which are divided into two different experimental sessions,** P<0.01 vs vehicle + vehicle; ^p<0.05 vs oxalyl platinum + vehicle.
Figure 29 shows the perimeter of the fibers. Data are reported as mean ± S.E.M. of the values obtained by 10 rats per group, which are divided into two different experimental sessions, *P<0.05 vs vehicle + vehicle; ^p<0.05 vs oxalyl platinum + vehicle.
Figure 30 reports the minimal Feret's diameter. Data are reported as mean ± S.E.M. of the values obtained by 10 rats per group, which are divided into two different experimental sessions,** P<0.01 vs vehicle + vehicle; ^p<0.05 vs oxalyl platinum + vehicle.
Figure 31 shows the diameter of the myotubes. Data are reported as mean ± S.E.M. of the values obtained by 10 rats per group, which are divided into two different experimental sessions, *P<0.05 vs vehicle + vehicle; ^p<0.05 vs oxalyl platinum + vehicle.
Figure 32 shows the number of nuclei per myotube. Data are reported as mean ± S.E.M. of the values obtained by 10 rats per group, which are divided into two different experimental sessions.
Figure 33 shows the evaluation of the muscle lipid peroxidation. At day 15 the gastrocnemius muscle has been explanted. The measurement of the oxidation state of the lipids has been carried out by means of the reaction with thiobarbituric acid and the results are expressed as TBARS amount (Thiobarbituric acid reactive substances.). Data are reported as mean ± S.E.M. of the values obtained by 10 rats per group, which are divided into two different experimental sessions,** P<0.01 vs vehicle + vehicle; ^P<0.01 vs oxalyl platinum + vehicle.
Figure 34 shows the evaluation of the muscle protein carbonylation. At day 15 the gastrocnemius muscle has been explanted. The measurement of the oxidation state of the proteins has been carried out by means of western blot after reacting the protein homogenate with DNPH (2-4-dinitrophenylhydrazine). For each sample the normalization with respect to the expression of the beta-actin reference protein has been carried out. Data are reported as mean ± S.E.M. of the values obtained by 10 rats per group, which are divided into two different experimental sessions,** P<0.01 vs vehicle + vehicle; ^P<0.01 vs oxalyl platinum + vehicle.
Figure 35 shows the evaluation of the enzyme activity of superoxide dismutase.
Figure 36 shows the evaluation of the enzyme activity of superoxide dismutase.
Figure 37 shows the evaluation of the muscle levels of NF-kB. At day 15 the gastrocnemius muscle has been explanted. The measurement of the oxidation state of the proteins has been carried out by western blot. For each sample the normalization with respect to the expression of the beta-actin reference protein has been carried out. Data are reported as mean ± S.E.M. of the values obtained by 10 rats per group, which are divided into two different experimental sessions,** P<0.01 vs vehicle + vehicle; ^p<0.05 vs oxalyl platinum + vehicle.

### Experimental Section

### Example 1

### Materials and Methods

### Experiment 1

### Animals

Sprague-Dawley male rats of about 200 g provided by Harlan Company, Italy have been used. The animals have been stabled in groups of four, in cages of 26x41 cm dimension at the Centro per la Stabulazione Animali da Laboratorio (Ce.S.A.L.) of the Università di Firenze with a 12 h circadian cycle with "ad libitum" water and food. The rats have been fed with standard diet and stabled at a temperature of 23±1°C. The experimental procedure has been approved by the local committee for the control of the experimentation on laboratory animals. All the experiments are carried out in accordance with legislations of the European Council of 24th November 1986 (DL 116/92; 86/609/EEC) concerning the manipulation of research animals and in agreement with the "National Institute of Health Guide for the Care and Use of Laboratory Animals", by acknowledge of the guidelines of the "International Association for the Study of Pain". All the necessary measurements have been taken to reduce at the minimum both the number of the animals and their discomfort.

### Model of muscle damage induced by dexamethasone

Dexamethasone has been intraperitoneally administrated to the rat at the daily dose of 600 microg/kg for 15 days, until an evident loss of body weight with respect to the control group treated with the vehicle.

### Evaluation of the muscle function and motor abilities

The grip force has been measured by the Grip Force test. The integrity of the motor coordination ability of the animal has been further analyzed with the aid of the Rota Rod instrument used at the speed of 10 revolutions per minute. The animal was placed on a rotating rod for 10 minutes, the number of falls and the overall time spent in balance have been measured. After a maximum of 6 falls of the animal the session has been considered completed.

### Irwin test

The test has been carried out in accordance with the work of Irwin to evaluate some behavioral parameters such as: behavior (spontaneous activity, passivity, curiosity, etc.), excitation of C.N.S. (tremors, convulsions, etc.), movements (ataxia, stereotypy, etc.), muscle tone (physical strength, etc.), reflexes, involuntary signs (piloerection, exophthalmia, salivation, etc.), toxicity (instant or delayed death). All the parameters have been evaluated within a range with limits of 0-4/4-0 (except for the measurement of the body temperature which is -4/+4 depending if it is hyperthermia or hypothermia).

### Tissue biopsies

At the end of the treatments the animals have been sacrificed and the gastrocnemius, tibialis anterior and extensor digitorum muscles have been removed. The tendons and the connective tissue were removed from the muscles and then the latter were analyzed.

### Lipoperioxidation (Thiobarbituric acid reactive substances, TBARS)

The tissue lysate has been added with 4 mL of 36 mM thiobarbituric acid (Sigma-Germany) (in a 10% acetic acid solution (Merck-Germany) brought to pH 4.0 with NaOH (Merck- Germany). The samples have been boiled for 1 hour. The reaction has been quenched by placing the vials into ice, the solution has been centrifuged for 10 minutes at 1.600 xg at 4°C. The fluorescence resulting from the reaction between malonyl dialdehyde and thiobarbituric acid has been read at an excitation wavelength of 530 nm and at an emission wavelength of 550 nm (Cayman Chemical Company-USA) in a Perkin-Elmer fluorescence reader.

### Protein oxidation, evaluation of the carbonylation level of the proteins

The tissue lysate has been lysate in a buffer made of 50 mM Tris-HCl pH 8.0, 150 nM NaCl, ImM EDTA, 0.5% Triton X-100, Complete Protease Inhibitor (Roche-Germany), and underwent a freezing/thawing cycle in order to complete the membrane lysis. The suspension has been centrifuged at 5000xg at 4°C, the supernatant solution has been stored and the protein dosage has been carried out thereon by means of the bicinchoninic acid assay (Sigma-Germany).

10 g of proteins have been denatured by adding SDS (Sigma-Germany) in order to obtain a 6% SDS final concentration. Then the samples have been derivatized by adding 10 mM DNPH (Sigma-Germany) (2-4-dinitrophenylhydrazine) and incubating at room temperature for 15 minutes. The proteins of each sample have been solved by 12% polyacrylamide gel electrophoresis (Bio-Rad-CA) and transferred on a nitrocellulose membrane (Bio-Rad-CA). After blocking the non-specific sites in a 1% albumin solution (Sigma-Germany) in phosphate buffer added with 0.1% Tween 20 (Sigma-Germany), the membrane has been incubated with an anti-DNPH antibody (1:5000; Chemicon International) overnight at 4°C. The incubation followed with an anti-Rabbit secondary antibody conjugated with a peroxidase (1:5000, Celi signaling-USA) for 1 hour at room temperature. The protein expression has been evaluated by the chemiluminescence technique (ECL; Pierce-USA); the densitometric analysis has been carried out by using the software for the image analysis "Scion Image". For each sample the normalization with respect to the expression of the beta-actin reference protein has been carried out.

### Example 2

### Experiment 2

### Cell cultures

Murine myoblast cultures (C2C12) have been obtained from American Type Culture Collection (Manassas, USA). The cells have been cultured in DMEM-high glucose containing 10% Fetal Bovine Serum (FBS), 2 mM glutamine, 100 U/mL penicillin and 100 µg/mL streptomycin. The cells have been stored in a humidified incubator at 37°C and with 5% CO2 (v/v). The myoblasts differentiation into myotubes has been induced after reaching the 80% cell confluency, by substituting FBS with 2% horse serum. After 7 days of incubation the culture showed the characteristic formation of myotubes.

### Pharmacological treatments

The myoblast cultures have been treated with Dexamethasone (Sigma - Italy), Loganin (Apharm, Italy), Paeoniflorin (Apharm, Italy), withania extract (Apharm, Italy), grape seed extract (Farmalabor, Italy) and calcium hydroxymethylbutyrate (HMB) (Barentz - Italy) at various concentrations depending on the different experimental requirements.

### Cell viability assay (MTT)

Murine myoblasts (C2C12) have been plated at 3000 cells/well in 96 well plates. Each well contained a volume of 200 microL of growth medium. After 48 hours, the cells have been treated depending on the different experimental requirements, by using white DMEM as vehicle. Concerning the model of cell damage induced by dexamethasone, the cells have been incubated with different concentrations of corticosteroid with or without the substances under examination. The latter have been tested both in combination and alone without the corticosteroid.

At the end of the different pharmacological treatments the cells have been washed with PBS and a solution of 3-(4,5-dimethylthiazol-2-yl)-2,5-diphenyltetrazolium bromide (MTT, Sigma-Germany) at a concentration of 1 mg/mL in white DMEM has been added to each well, the cells have been incubated for 1 hour at 37°C by such a solution. At the end of the incubation the MTT solution has been drawn and 100 microL of DMSO (Sigma - Germany) have been added to each well in order to solubilize the formazan crystals formed in the mitochondria of the vital cells. The absorbance of the so obtained violet solutions has been measured at the wavelength of 550 nm.

### Example 3

### Experiment 3

### Animals

Sprague-Dawley male rats of about 200 g provided by Harlan Company, Italy. The animals have been stabled in groups of four, in cages of 26x41 cm dimension at the Centro per la Stabulazione Animali da Laboratorio (Ce.S.A.L.) of the Universita di Firenze with a 12 h circadian cycle with "ad libitum" water and food. The rats have been fed with standard diet and stabled at a temperature of 23±1°C. The experimental procedure has been approved by the local committee for the control of the experimentation on laboratory animals. All the experiments have been carried out in accordance with legislations of the European Council of 24th November 1986 (DL 116/92; 86/609/EEC) concerning the manipulation of research animals and in agreement with the "National Institute of Health Guide for the Care and Use of Laboratory Animals", by acknowledge of the guidelines of the "International Association for the Study of Pain". All the necessary measurements have been taken to reduce at the minimum both the number of the animals and their discomfort.

### Model of muscle damage induced by dexamethasone

Dexamethasone has been intraperitoneally administrated to the rat at the daily dose of 600 µg/kg for 15 days (Yamamoto et al., Muscle and Nerve, 41:819-827, 2010).

### Behavioral evaluations

The motor abilities of the animals have been evaluated as spontaneous activity, stretching ability of the paws and climbing in accordance with the method described by Garcia et al. (Garcia JH, Wagner S, Liu KF, Hu XJ. Neurological deficit and extent of neuronal necrosis attributable to middle cerebral artery occlusion in rats. Statistical validation. Stroke 1995b;26:627-34). The grip force has been measured by the Grip Force test (Ugo Basile, Italy). The integrity of the motor coordination ability of the animal has been further analyzed with the aid of the Rota Rod instrument (Ugo Basile, Varese, Italy) used at the speed of 10 revolutions per minute. The animal was placed on a rotating rod for 10 minutes, the number of falls and the overall time spent in balance have been measured. After a maximum of 6 falls the animal completed the session (Vaught et al., Neuropharmacology, 4: 211-216, 1985).

### Tissue biopsies

At the end the treatments the animals have been sacrificed and the gastrocnemius and extensor digitorum muscles have been removed. The tendons and the connective tissue were removed from the muscles and then the latter were analyzed.

### Histological analysis

The tissues have been fixed in 4% paraformaldehyde then dehydrated and included in paraffin. 10 µm sections have been obtained and stained with a mixture of hematoxylin/eosin. The whole cross section of the muscle has been measured. The morphometric and histological analysis have been carried out on digitalized images obtained with a 40x microscope. The measurements will be carried out on three different areas of the same section (50-75% of the total surface of the section).

### Immunofluorescence

In order to highlight the actin fibers, phalloidin-labeled TRITC (1:40; Life Technologies, Italy) has been used, 4',6-diamidino-2-phenylindole dihydrochloride (DAPI, 1:2000; Sigma- Aldrich, Milan, Italy) to detect the nuclei. The microscope slides have been mounted with ProLong (Life Technologies, Milan, Italy) and observed with a Leica DM6000B microscope (Leica, Mannheim, Germany). The quantitative analysis has been carried out by using the following software: "cell counter" plugin of ImageJ; FIJI software (ImageJ, NIH, Bethesda, Maryland, USA); "Moments" algorithm.

### Morphometric measurements

Following the hematoxylin---eosin staining or a fluorescent labelling with phalloidin the morphometric measurements have been carried out by using full cross sections (ImageJ software). Up to 3000 fibers per muscle per section have been analyzed. The area of the fiber, the number for optical field, the perimeter, the minimum Feret's diameter (Briguet et al., Neuromuscolar Dis, 2004, 14:675--682) have been determined.

### Lipofuscin levels

Muscle tissue sections of 200 mg will be homogenized in a chloroform/methanol (2:1, v/v) solution containing 0.005% butylhydroxytoluol (w/v). After centrifugation at 1000 x g for 0 minutes the emitted fluorescence will be read by using an emission wavelength of 450 and an excitation wavelength of 350 nm. (Faist V, Koenig J, Hoeger H, Elmadfa I. Mitochondrial oxygen consumption, lipid peroxidation and antioxidant enzyme systems in skeletal muscle of senile dystrophic mice. Pflugers Arch. 1998 Dec;437(1):168-71; Jung T, Höhn A, Grune T. Lipofuscin: detection and quantification by microscopic techniques. Methods Mol Biol. 2010;594:173-93).

### Example 4

### Experiment 4

### Animals

Sprague-Dawley male rats of about 200 g provided by Harlan Company, Italy have been used. The animals have been stabled in groups of four, in cages of 26x41 cm dimension at the Centro per la Stabulazione Animali da Laboratorio (Ce.S.A.L.) of the Università di Firenze with a 12 h circadian cycle with "ad libitum" water and food. The rats have been fed with standard diet and stabled at a temperature of 23±1°C. The experimental procedure has been approved by the local committee for the control of the experimentation on laboratory animals. All the experiments are carried out in accordance with legislations of the European Council of 24th November 1986 (DL 116/92; 86/609/EEC) concerning the manipulation of research animals and in agreement with the "National Institute of Health Guide for the Care and Use of Laboratory Animals", by acknowledge of the guidelines of the "International Association for the Study of Pain". All the necessary measurements have been taken to reduce at the minimum both the number of the animals and their discomfort.

### Model of muscle damage induced by oxalyl platinum

Oxalyl platinum has been intraperitoneally administrated to the rat at the daily dose of 2.4 mg/kg for 15 days (Cavaletti et al., Eur J Cancer, 2001; Di Cesare Mannelli et al., Exp Neurol, 2014).

### Evaluation of the muscle function and motor abilities

The grip force has been measured by the Grip Force test (Ugo Basile, Italy). The integrity of the motor coordination ability of the animal has been further analyzed with the aid of the Rota Rod instrument (Ugo Basile, Varese, Italy) used at the speed of 10 revolutions per minute. The animal was placed on a rotating rod for 10 minutes, the number of falls and the overall time spent in balance have been measured. After a maximum of 6 falls of the animal the session has been considered completed (Vaught et al., Neuropharmacology, 4: 211-216, 1985).

### Irwin test

The test has been carried out in accordance with the work of Irwin (Psychopharmacologia 13: 222-257; 1968) to evaluate some behavioral parameters such as: behavior (spontaneous activity, passivity, curiosity, etc.), excitation of C.N.S. (tremors, convulsions, etc.), movements (ataxia, stereotypy, etc.), muscle tone (physical strength, etc.), reflexes, involuntary signs (piloerection, exophthalmia, salivation, etc.), toxicity (instant or delayed death). All the parameters have been evaluated within a range with limits of 0-4/4-0 (except for the measurement of the body temperature which is -4/+4 depending if it is hyperthermia or hypothermia).

### Tissue biopsies

At the end of the treatments the animals have been sacrificed and the gastrocnemius, tibialis anterior and extensor digitorum muscles have been removed. The tendons and the connective tissue were removed from the muscles and then the latter were analyzed.

### Histological analysis

The tissues have been fixed in 4% paraformaldehyde then dehydrated and included in paraffin. 10 µm sections have been obtained and stained with a mixture of hematoxylin/eosin. The whole cross section of the muscle has been measured. The morphometric and histological analysis have been carried out on digitalized images obtained with a 40x microscope. The measurements will be carried out on three different areas of the same section (50-75% of the total surface of the section).

### Immunofluorescence

In order to highlight the actin fibers, phalloidin-labeled TRITC (1:40; Life Technologies, Italy) has been used, 4',6-diamidino-2-phenylindole dihydrochloride (DAPI, 1:2000; Sigma- Aldrich, Milan, Italy) to reveal the nuclei. The microscope slides have been mounted with ProLong (Life Technologies, Milan, Italy) and observed with a Leica DM6000B microscope (Leica, Mannheim, Germany). The quantitative analysis has been carried out by using the following software: "cell counter" plugin of ImageJ; FIJI software (ImageJ, NIH, Bethesda, Maryland, USA); "Moments" algorithm.

### Morphometric measurements

Following the hematoxylin-eosin staining or a fluorescent labelling with phalloidin the morphometric measurements have been carried out by using full cross sections (ImageJ software). Up to 3000 fibers per muscle per section have been analyzed. The area of the fiber, the number for optical field, the perimeter, the minimum Feret's diameter (Briguet et al., Neuromuscolar Dis, 2004, 14:675-682) have been determined.

### Lipoperioxidation (Thiobarbituric acid reactive substances, TBARS)

The tissue lysate has been added with 4 mL of 36 mM thiobarbituric acid (Sigma-Germany) (in a 10% acetic acid solution (Merck-Germany) brought to pH 4.0 with NaOH (Merck- Germany). The samples have been boiled for 1 hour. The reaction has been quenched by placing the vials into ice, the solution has been centrifuged for 10 minutes at 1.600 xg at 4°C. The fluorescence resulting from the reaction between malonyl dialdehyde and thiobarbituric acid has been read at an excitation wavelength of 530 nm and at an emission wavelength of 550 nm (Cayman Chemical Company-USA) in a Perkin-Elmer fluorescence reader.

### Protein oxidation, evaluation of the carbonylation level of the proteins

The tissue lysate has been lysate in a buffer made of 50 mM Tris-HCl pH 8.0, 150 nM NaCl, ImM EDTA, 0.5% Triton X-100, Complete Protease Inhibitor (Roche-Germany), and underwent a freezing/thawing cycle in order to complete the membrane lysis. The suspension has been centrifuged at 5000xg at 4°C, the supernatant solution has been stored and the protein dosage has been carried out thereon by means of the bicinchoninic acid assay (Sigma - Germany). 10 µg of proteins have been denatured by adding SDS (Sigma - Germany) in order to obtain a 6% SDS final concentration. Then the samples have been derivatized by adding 10 mM DNPH (Sigma - Germany) (2-4-dinitrophenylhydrazine) and incubating at room temperature for 15 minutes. The proteins of each sample have been solved by 12% polyacrylamide gel electrophoresis (Bio-Rad -CA) and transferred on a nitrocellulose membrane (Bio-Rad-CA). After blocking the non-specific sites in a 1% albumin solution (Sigma-) in phosphate buffer added with 0.1% Tween 20 (Sigma - Germany), the membrane has been incubated with an anti-DNPH antibody (1:5000; Chemicon International) overnight at 4°C. The incubation followed with an anti-Rabbit secondary antibody conjugated with a peroxidase (1:5000, Cell signaling-USA) for 1 hour at room temperature. The protein expression has been evaluated by the chemiluminescence technique (ECL; Pierce-USA); the densitometric analysis has been carried out by using the software for the image analysis "Scion Image". For each sample the normalization with respect to the expression of the □ actin reference protein has been carried out.

### Enzyme activity and amino acids levels

The superoxide dismutase activity has been determined by means of the xanthinexanthine oxidase reaction (Ding et al., Mol Nutr Food Res. 2013 Feb;57(2):365-9). The creatine kinase levels have been dosed by means of the enzymatic analysis tracking nicotinamide adenine diphesphopyridine - NADPH). Testosterone has been determined by means of ELISA method. The plasma and muscle levels of glutamine have been determined by enzyme reaction based on the hydrolysis of glutamine to glutamate.

### Western Blot

The tissue has been homogenized in a lysis buffer composed of 50 mM Tris-HCl pH 8.0, 150 mM NaCl, 1 mM EDTA, 0.5% Triton X-100, Complete Protease Inhibitor (Roche). The homogenate has been incubated for 30 minutes in ice, then sonicated with 3 pulses for 10 seconds and centrifugated for 15 min. at 13000xg at 4°C. The proteins present in the supernatant have been determined by means of bicinchoninic acid assay. 30 µg of such proteins per each sample have been separated by electrophoresis on a 4 to 12% polyacrylamide gradient and transferred on a nitrocellulose membrane. After blocking the non-specific binding sites, the membrane has been incubated with a polyclonal antibody specific to NFkB for 16 h at 4°C; after that incubation followed for 1 h at room temperature with a secondary antibody conjugated with a peroxidase. The protein expression has been detected by means of chemiluminescence technique. The densitometric analysis has been carried out by using the image analysis software "Scion Image". For each sample the normalization has been carried out by Beta-actin.

### Example 5

### Composition according to the invention

A composition in the form of granules is prepared, comprising
- 1 g of calcium 3-hydroxy-3-methylbutanoate;
- 150 mg of grape seed extract;
- 300 mg of withania extract, advantageously withania somnifera;
- 3 g of ornithine alpha-ketoglutarate;
together with conventional excipients and vehicles.

### Example 6

### Composition according to the invention

A composition in the powder form is prepared, comprising
- 1 g of calcium 3-hydroxy-3-methylbutanoate;
- 150 mg of grape seed extract;
- 300 mg of withania extract, advantageously withania somnifera;
- 3 g of ornithine alpha-ketoglutarate;
together with conventional excipients and vehicles.

### Example 7

- 1 g of 3-hydroxy-3-methylbutanoic acid;
- 150 mg of grape seed extract;
- 300 mg of withania extract, advantageously withania somnifera;
- 3 g of ornithine alpha-ketoglutarate;
together with conventional excipients and vehicles.

### Example 8

### Composition according to the invention

A composition in the powder form is prepared, comprising
- 1 g of 3-hydroxy-3-methylbutanoate calcium salt;
- 150 mg of grape seed extract;
- 300 mg of withania extract, advantageously withania somnifera;
- 3 g of ornithine alpha-ketoglutarate;
together with conventional excipients and vehicles.

### Example 9

### Composition according to the invention

A composition in the powder form is prepared, comprising
- 1.5 g of calcium 3-hydroxy-3-methylbutanoate;
- 250 mg of grape seed extract;
- 250 mg of withania extract, advantageously withania somnifera;
- 3.5 g of ornithine alpha-ketoglutarate;
together with conventional excipients and vehicles.

### Example 10

### Composition according to the invention

A composition in the powder form is prepared, comprising
- 1.5 g of calcium 3-hydroxy-3-methylbutanoate;
- 250 mg of grape seed extract;
- 250 mg of withania extract, advantageously withania somnifera;
- 3.5 g of ornithine alpha-ketoglutarate;
- 10.3 g of a mixture of L-leucine, L-valine, L-isoleucine, L-histidine, L-lysine, L-methionine, L-cystine or L-cysteine, L-phenylalanine, L-tyrosine, L-threonine, L-glutamine, L-arginine and L-tryptophan;
- Vitamin B6;
- Vitamin D3;
- Creatine;
together with excipients and additives among which maltodextrins, hydroxypropyl methylcellulose, sucrose esters, citric acid, sucralose flavorings and silicon dioxide.

### Example 11

### Composition according to the invention

A composition in the powder form is prepared, comprising
- 1.5 g of calcium 3-hydroxy-3-methylbutanoate;
- 250 mg of grape seed extract;
- 250 mg of withania extract, advantageously withania somnifera;
- 3.5 g of ornithine alpha-ketoglutarate;
- 10.3 g of a mixture of L-leucine, L-valine, L-isoleucine, L-histidine, L-lysine, L-methionine, L-cystine or L-cysteine, L-phenylalanine, L--tyrosine, L-threonine, L-glutamine, L-arginine and L-tryptophan;
- 1.5 mg of Vitamin B6;
- 20 microg of Vitamin D3;
- 1500 mg of Creatine;
maltodextrins, hydroxypropyl methylcellulose, sucrose esters, citric acid, flavorings, sugar substitutes and anti-caking agents.

## Claims

1. A combination of active ingredients comprising 3-hydroxy-3-methylbutanoic acid or a pharmaceutically acceptable salt thereof, a grape seed extract and a withania extract.

2. The combination according to claim 1, **characterized in that** said pharmaceutically acceptable salt of 3-hydroxy-3-methylbutanoic acid is the calcium salt.

3. The combination according to claim 1, **characterized in that** it further comprises ornithine alpha-ketoglutarate.

4. The combination according to claim 2 or 3, **characterized in that** it is a fixed combination of calcium 3-hydroxy-3-methylbutanoate/grape seed extract/withania extract/ornithine alpha-ketoglutarate at a reciprocal weight ratio of 1/0.1-0.30/0.1-0.5/2-5 respectively.

5. The combination according to claim 4, **characterized in that** it is a fixed combination of calcium 3-hydroxy-3-methylbutanoate/grape seed extract/withania extract/ornithine alpha-ketoglutarate at a reciprocal weight ratio of 1/0.15/0.30/3 respectively or of 1/0.16/0.16/2.3 respectively.

6. A pharmaceutical or nutraceutical composition comprising the combination according to any one of claims 1 to 5, together with conventional carriers and/or excipients.

7. The composition according to claim 6, in the form of dose unit, comprising
- 1 to 4 g of 3-hydroxy-3-methylbutanoic acid;
- 100 to 500 mg of grape seed extract;
- 100 to 1000 mg of withania extract, advantageously withania somnifera; and
- 1 to 10 g of ornithine alpha-ketoglutarate,
together with conventional vehicles and/or excipients.

8. The composition according to claim 7 comprising
- 1 g of calcium 3-hydroxy-3-methylbutanoate;
- 150 mg of grape seed extract;
- 300 mg of withania extract, advantageously withania somnifera; and
- 3 g of ornithine alpha-ketoglutarate;
together with conventional vehicles and/or excipients.

9. The composition according to claim 7 comprising
- 1.5 g of calcium 3-hydroxy-3-methylbutanoate;
- 250 mg of grape seed extract;
- 250 mg of withania extract, advantageously withania somnifera; and
- 3.5 g of ornithine alpha-ketoglutarate;
together with conventional vehicles and/or excipients.

10. The composition according to any one of claims 6 to 9, **characterized in that** it is a solid oral composition, preferably in form of granules, powder or gel, or a gel.

11. The composition according to any one of claims 6 to 10, **characterized in that** it further comprises amino acids and/or vitamins, preferably Vitamin B6 and/or Vitamin D3.

12. The composition according to claim 11, **characterized in that** it comprises one or more amino acids selected from L-leucine, L-valine, L-isoleucine, L-histidine, L-lysine, L-methionine, L-cystine, L-cysteine, L-phenylalanine, L-tyrosine, L-threonine, L-glutamine, L-arginine and L-tryptophan.

13. The composition according to any one of claims 11 and 12, **characterized in that** it further comprises creatine.

14. The composition according to any one of claims 6 to 13 for its use in the treatment and/or prevention of sarcopenia and/or as anabolic steroid.

15. The composition for the use according to claim 14, to increase the muscle mass during training or in elderly and debilitated people and in people who have lost musculature due to inactivity or bedriddening; in the cachexia states; to protect the muscle from the damages induced by intense workout and to contrast fatigue feeling and/or to improve physical performances.

## Patentansprüche

1. Eine Kombination von Wirkstoffen, umfassend 3-Hydroxy-3-methylbutansäure oder ein pharmazeutisch verträgliches Salz davon, einen Traubenkernextrakt und einen Withania-Extrakt.

2. Die Kombination gemäß Anspruch 1, **dadurch gekennzeichnet, dass** das pharmazeutisch verträgliche Salz der 3-Hydroxy-3-methylbutansäure das Calciumsalz ist.

3. Die Kombination gemäß Anspruch 1, **dadurch gekennzeichnet, dass** sie ferner Ornithin-alpha-ketoglutarat umfasst.

4. Kombination gemäß Anspruch 2 oder 3, **dadurch gekennzeichnet, dass** es sich um eine feste Kombination von Calcium-3-hydroxy-3-methylbutanoat/Traubenkernextrakt/Withaninextrakt/ Ornithin-alpha-ketoglutarat bei einem gegenseitigen Gewichtsverhältnis von 1/0,1-0,30/0,1-0,5/2-5 handelt.

5. Die Kombination gemäß Anspruch 4, **dadurch gekennzeichnet, dass** es sich um eine feste Kombination von Calcium-3-hydroxy-3-methylbutanoat/ Traubenkernextrakt/Withaninextrakt/Ornithin-alpha-ketoglutarat bei einem gegenseitigen Gewichtsverhältnis von 1/0,15/0,30/3 oder von 1/0,16/0,16/2,3 handelt.

6. Eine Pharmazeutische oder nutrazeutische Zusammensetzung, umfassend die Kombination gemäß einem der Ansprüche 1 bis 5 zusammen mit herkömmlichen Trägern und/oder Hilfsstoffen.

7. Die Zusammensetzung gemäß Anspruch 6 in Form einer Dosiseinheit, umfassend
1 bis 4 g 3-Hydroxy-3-methylbutansäure;
100 bis 500 mg Traubenkernextrakt;
100 bis 1000 mg Withania-Extrakt, vorteilhafterweise Withania somnifera; und
1 bis 10 g Ornithin-alpha-ketoglutarat,
zusammen mit herkömmlichen Trägern und/oder Hilfsstoffen.

8. Die Zusammensetzung gemäß Anspruch 7, umfassend
1 g Calcium-3-hydroxy-3-methylbutanoat;
150 mg Traubenkernextrakt;
300 mg Withania-Extrakt, vorteilhafterweise Withania somnifera; und
3 g Ornithin-Alpha-Ketoglutarat;
zusammen mit herkömmlichen Trägern und/oder Hilfsstoffen.

9. Die Zusammensetzung gemäß Anspruch 7 umfassend
- 1,5 g Calcium-3-hydroxy-3-methylbutanoat;
- 250 mg Traubenkernextrakt;
- 250 mg Withania-Extrakt, vorteilhafterweise Withania somnifera; und
- 3,5 g Ornithin-Alpha-Ketoglutarat;
zusammen mit herkömmlichen Trägern und/oder Hilfsstoffen.

10. Die Zusammensetzung gemäß einem der Ansprüche 6 bis 9, **dadurch gekennzeichnet, dass** es sich um eine feste orale Zusammensetzung, vorzugsweise in Form von Granulat, Pulver oder Gel, oder um ein Gel handelt.

11. Die Zusammensetzung gemäß einem der Ansprüche 6 bis 10, **dadurch gekennzeichnet, dass** sie ferner Aminosäuren und/oder Vitamine umfasst, vorzugsweise Vitamin B6 und/oder Vitamin D3.

12. Die Zusammensetzung gemäß Anspruch 11, **dadurch gekennzeichnet, dass** sie eine oder mehrere Aminosäuren umfasst, ausgewählt aus L-Leucin, L-Valin, L-Isoleucin, L-Histidin, L-Lysin, L-Methionin, L-Cystin, L-Cystein, L-Phenylalanin, L-Tyrosin, L-Threonin, L-Glutamin, L-Arginin und L-Tryptophan.

13. Die Zusammensetzung gemäß einem der Ansprüche 11 und 12, **dadurch gekennzeichnet, dass** sie ferner Kreatin umfasst.

14. Die Zusammensetzung gemäß einem der Ansprüche 6 bis 13 zur Verwendung bei der Behandlung und/oder Vorbeugung von Sarkopenie und/oder als anaboles Steroid.

15. Die Zusammensetzung zur Verwendung gemäß Anspruch 14 zur Erhöhung der Muskelmasse während des Trainings oder bei älteren und geschwächten Menschen und bei Menschen, die aufgrund von Inaktivität oder Bettlägerigkeit Muskulatur verloren haben; in den Kachexiezuständen; um den Muskel vor Schäden durch intensives Training zu schützen und Ermüdungsgefühlen entgegenzuwirken und/oder die körperliche Leistungsfähigkeit zu verbessern.

## Revendications

1. Combinaison de principes actifs comprenant de l'acide 3-hydroxy-3-méthylbutanoïque ou un sel pharmaceutiquement acceptable de celui-ci, un extrait de pépins de raisin et un extrait de withania.

2. Combinaison selon la revendication 1, **caractérisée en ce que** ledit sel pharmaceutiquement acceptable de l'acide 3-hydroxy-3-méthylbutanoïque est le sel de calcium.

3. Combinaison selon la revendication 1, **caractérisée en ce qu'**elle comprend en outre de l'alpha-cétoglutarate d'ornithine.

4. Combinaison selon la revendication 2 ou 3, **caractérisée en ce qu'**il s'agit d'une combinaison fixe de 3-hydroxy-3-méthylbutanoate de calcium/ extrait de pépins de raisin/ extrait de withania/ alpha-cétoglutarate d'ornithine à un rapport pondéral réciproque de 1/0,1 - 0,30/0,1 - 0,5/2 - 5 respectivement.

5. Combinaison selon la revendication 4, **caractérisée en ce qu'**il s'agit d'une combinaison fixe de 3-hydroxy-3-méthylbutanoate de calcium/ extrait de pépins de raisin/ extrait de withania/ alpha-cétoglutarate d'ornithine à un rapport pondéral réciproque de 1/0,15/0,30/3 respectivement ou de 1/0,16/0,16/2,3 respectivement.

6. Composition pharmaceutique ou nutraceutique comprenant la combinaison selon l'une quelconque des revendications 1 à 5, ainsi que des supports et/ou excipients classiques.

7. Composition selon la revendication 6, sous forme d'unité de dose, comprenant
- 1 à 4 g d'acide 3-hydroxy-3-méthylbutanoïque,
- 100 à 500 mg d'extrait de pépins de raisin ;
- 100 à 1000 mg d'extrait de withania, avantageusement de withania somnifera ; et
- 1 à 10 g d'alpha-cétoglutarate d'ornithine,
ainsi que des véhicules et/ou excipients classiques.

8. Composition selon la revendication 7 comprenant
- 1 g de 3-hydroxy-3-méthylbutanoate de calcium ;
- 150 mg d'extrait de pépins de raisin ;
- 300 mg d'extrait de withania, avantageusement de withania somnifera ; et
- 3 g d'alpha-cétoglutarate d'ornithine ;
ainsi que des véhicules et/ou excipients classiques.

9. Composition selon la revendication 7 comprenant
- 1,5 g de 3-hydroxy-3-méthylbutanoate de calcium ;
- 250 mg d'extrait de pépins de raisin ;
- 250 mg d'extrait de withania, avantageusement de withania somnifera ; et
- 3,5 g d'alpha-cétoglutarate d'ornithine ;
ainsi que des véhicules et/ou excipients classiques.

10. Composition selon l'une quelconque des revendications 6 à 9, **caractérisée en ce qu'**elle est une composition orale solide, de préférence sous forme de granulés, de poudre ou de gel, ou un gel.

11. Composition selon l'une quelconque des revendications 6 à 10, **caractérisée en ce qu'**elle comprend en outre des acides aminés et/ou des vitamines, de préférence la vitamine B6 et/ou la vitamine D3.

12. Composition selon la revendication 11, **caractérisée en ce qu'**elle comprend un ou plusieurs acides aminés choisis parmi la L-leucine, la L-valine, la L-isoleucine, la L-histidine, la L-lysine, la L-méthionine, la L-cystine, la L-phénylalanine, la L-tyrosine, la L-thréonine, la L-glutamine, la L-arginine et le L-tryptophane.

13. Composition selon l'une quelconque des revendications 11 et 12, **caractérisée en ce qu'**elle comprend en outre de la créatine.

14. Composition selon l'une quelconque des revendications 6 à 13 pour son utilisation dans le traitement et/ou la prévention de la sarcopénie et/ou comme stéroïde anabolisant.

15. Composition pour l'utilisation selon la revendication 14, pour augmenter la masse musculaire pendant l'entraînement ou chez les personnes âgées et affaiblies et chez les personnes qui ont perdu leur musculature en raison de l'inactivité ou de l'alitement ; dans les états de cachexie ; pour protéger le muscle des dommages induits par un entraînement intense et pour contraster la sensation de fatigue et/ou pour améliorer les performances physiques.
